# EUROPÄISCHE PATENTSCHRIFT

(11) **EP 2 787 989 B1**
(45) Veröffentlichungstag und Bekanntmachung des Hinweises auf die Patenterteilung: **06.04.2016**
(21) Anmeldenummer: 12786848.7
(22) Anmeldetag: 10.11.2012
(51) Int. Cl.: A61K 8/35, A61K 31/343, C07D 307/93, A61K 8/49, A61K 31/34, A61Q 17/04, A61Q 19/04

(54) **GLUCURONOLACTONDERIVATE ALS SELBSTBRÄUNUNGSSUBSTANZEN**
GLUCURONOLACTONIC DERIVATIVES AS SELF TANNING AGENTS
DÉRIVÉS DE GLUCURONOLACTON EN TANT QU'AGENTS AUTOBRONZANTS

(30) Priorität: 08.12.2011 EP 11009713
(43) Veröffentlichungstag der Anmeldung: 15.10.2014
(73) Patentinhaber: Merck Patent GmbH, 64293 Darmstadt (DE)
(72) Erfinder: CAROLA, Christophe, 64625 Bensheim (DE); MUELLER, Tatjana, 64846 Gross-Zimmern (DE)
(86) Internationale Anmeldenummer: PCT/EP2012/004678
(87) Internationale Veröffentlichungsnummer: WO 2013/083225

(56) Entgegenhaltungen:
- WO-A1-95/22960
- WO-A1-2009/030372
- FR-A1- 2 717 176

## Beschreibung

Die Erfindung betrifft die Verwendung spezieller Glucuronolactonderivate als Selbstbräunungssubstanzen, als Bräunungsverstärker für Dihydroxyaceton oder für eine Mischung von Selbstbräunungssubstanzen enthaltend Dihydroxyaceton, zur Modulation des Farbtones, der bei der Bräunung mit Dihydroxyaceton oder durch die Mischung oder Zubereitung enthaltend Dihydroxyaceton erzielt wird, als Kontrastreduktionsmittel oder zur Färbung keratinhaltigen Fasern sowie Zubereitungen oder Mittel zur Färbung keratinhaltiger Fasern enthaltend diese speziellen Glucuronolactonderivate.

Der Trend weg von der vornehmen Blässe hin zur "gesunden, sportlich braunen Haut" ist seit Jahren ungebrochen. Um einen gebräunten Teint zu erzielen, setzen die Menschen ihre Haut der Sonnenstrahlung aus, da diese eine Pigmentierung durch eine Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden bei übermäßiger Bestrahlung mit Licht aus dem UVB-Bereich (Wellenlänge 280-320 nm) auf, wie beispielsweise ein erhöhtes Risiko, an Hautkrebs zu erkranken. Die übermäßige Einwirkung der UVB- und UVA-Strahlung (Wellenlänge: 320-400 nm) erzeugt hochreaktive Radikalspezies, die sich auch nach Beendigung der Bestrahlung weiter vermehren und als deren Folge es zu Faltenbildung und Hautalterung kommt.

Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Hornzellen) transportiert und dort als braune Hautfarbe sichtbar wird. Melanin schützt die Zellkerne vor weiterer Bestrahlung und den davon verursachten negativen Auswirkungen auf die Zell-DNA. Es legt sich wie ein Sonnenschirm um den Zellkern und schützt diesen so vor schädigender UV-Strahlung.

Je nach chemischer Zusammensetzung der biochemisch gebildeten Pigmente wir zwischen dem bräunlich-schwarzen Eumelanin und dem rötlich-gelben Pheomelanin unterschieden. Der beobachtete Hautfarbton wird vom Verhältnis dieser beiden Melaninarten bestimmt.

Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen. Bei der "Direkt-Pigmentierung", die mit der Sonnenbestrahlung einsetzt, werden vorwiegend farblose Melanin-Vorstufen durch UVA-Strahlung zu dunkel gefärbtem Melanin oxidiert. Da diese Oxidierung reversibel ist, führt sie zu einer nur kurz anhaltenden Hautbräunung.

Weitaus beliebter jedoch ist die künstliche Bräunung der Haut, welche sich durch Auftragen von sogenannten Selbstbräunern erzielen lässt, z.B offenbart in WO 2009/030372 A1 oder WO 95/22960 A1.

Die klassischen Selbstbräuner, wie beispielsweise 1,3-Dihydroxaceton (DHA), können mit den Proteinen und Aminosäuren der Hornschicht der Haut im Sinne einer Maillard-Reaktion oder über eine Michael Addition umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt.

Bei DHA handelt es sich um einen wasserlöslichen kristallinen Feststoff, der unter neutralen bis basischen Bedingungen nicht stabil ist. Diese Instabilität geht auch mit der Entwicklung kosmetisch unerwünschter Fehlgerüche einher.

Ein Problem, welches bei der Anwendung mit Selbstbräunersubstanzen, insbesondere mit 1,3-Dihydroxyaceton, häufig auftreten kann, ist, dass die Bräunung der Haut durch die Dominanz des Gelbanteils nach orange verfärbt wird.

Auch besteht weiterhin ein Bedarf nach dermatologisch verträglichen hautfärbenden Substanzen, insbesondere für die Kombination mit Dihydroxyaceton, die sich zum Einsatz in kosmetischen und/oder dermatologischen Zubereitungen oder Medizinprodukten eignen.

Die vorliegende Erfindung beschäftigt sich mit der Aufgabe, das Färben proteinhaltiger Matrices, insbesondere die Färbung der Haut hinsichtlich eines natürlicheren Farbtons, zu verbessern.

Die vorliegende Erfindung beschäftigt sich ebenfalls mit der Aufgabe, verträgliche, insbesondere hautverträgliche Farbstoffe zur Färbung keratinhaltiger Fasern zu entwickeln.

Es wurde überraschenderweise gefunden, dass die Glucuronolactonderivate der Formel I, wie nachfolgend beschrieben, und/oder deren Salze, Tautomere, Stereoisomere und/oder Solvate alleine in der Lage sind, die Haut zu färben, sowie zusammen mit Dihydroxyaceton die Haut dunkler einzufärben, als der Selbstbräuner Dihydroxyaceton allein und/oder zusammen mit Dihydroxyaceton eine Modulierung des Farbtons zu einem natürlicheren Farbton zu erreichen, in der Lage sind, den Kontrast zwischen stärker und weniger stark gefärbten Hautpartien herabzusetzen und sich auch sehr gut als direktziehende Farbstoffe für die Färbung von keratinhaltigen Fasern eignen. Diese Eigenschaft ist insofern überraschend, da das anomere C-Atom der Glucuronolactonderivate der Formel I geschützt vorliegt und somit keine Maillard-Reaktion im klassischen Sinne vorliegen kann.

Ein Gegenstand der Erfindung ist demzufolge die Verwendung mindestens einer Verbindung der Formel I 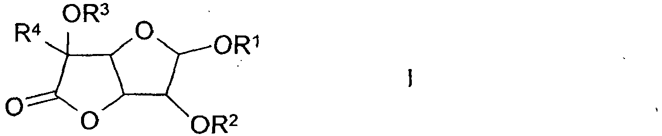
wobei R¹ und R² jeweils unabhängig voneinander Aryl,
eine geradkettige oder verzweigte Alkylgruppe mit 1 bis 30 C-Atomen, die mit Aryl substituiert sein kann oder
eine geradkettige oder verzweigte Alkenylgruppe mit 2 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
   a) durch mindestens eine geradkettige oder verzweigte Alkylgruppe mit 1 bis 30 C-Atomen und/oder
   b) durch mindestens eine Arylgruppe mit 6 bis 12 C-Atomen und/oder
   c) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
   Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
   Alkyl eine geradkettige oder verzweigte Alkylgruppe mit 1 bis 30 C-Atomen bedeutet,
   und/oder deren Salze, Tautomere, Stereoisomere und/oder Solvate, einschließlich deren Mischungen in allen Verhältnissen als Selbstbräunungssubstanz.

Ein weiterer Gegenstand der Erfindung ist demzufolge die Verwendung mindestens einer Verbindung der Formel I, wie zuvor beschrieben, und/oder deren Salze, Tautomere, Stereoisomere und/oder Solvate, einschließlich deren Mischungen in allen Verhältnissen als Bräunungsverstärker für Dihydroxyaceton oder für eine Mischung von Selbstbräunungssubstanzen enthaltend Dihydroxyaceton.

Ein weiterer Gegenstand der Erfindung ist demzufolge die Verwendung mindestens einer Verbindung der Formel I, wie zuvor beschrieben, und/oder deren Salze, Tautomere, Stereoisomere und/oder Solvate, einschließlich deren Mischungen in allen Verhältnissen zur Modulation des Farbtones, der bei der Bräunung mit Dihydroxyaceton oder durch die Mischung oder Zubereitung enthaltend Dihydroxyaceton erzielt wird.

Im Rahmen der Erfindung sind die Verbindungen der Formel I, so definiert, dass man darunter auch pharmazeutisch oder kosmetisch verwendbare Salze, Hydrate, Solvate, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Die Verbindungen der Formel I können ein oder mehrere chirale Zentren besitzen und daher in verschiedenen stereoisomeren Formen vorkommen. Die Formel I schließt alle diese Formen ein. Sofern als Ausgangsverbindung D-Glucuronsäure-3,6-lacton eingesetzt wird, so besitzen die Verbindungen der Formel I, wie zuvor beschrieben, hauptsächlich die Stereochemie, wie in Formel I-1 dargestellt, wobei R¹, R², R³ und R⁴ eine zuvor angegebene Bedeutung haben.

Die Verbindungen der Formel 1-1 sind Diastereomere, basierend auf α-D-(+)-Glucuronsäure-3,6-lacton und β-D-(+)-Glucuronsäure-3,6-lacton:

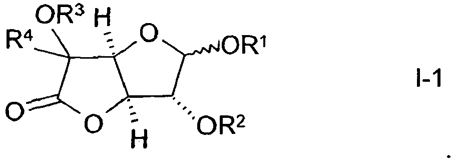

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern.

Andererseits können Verbindungen der Formel I mit Basen (z.B. Natrium- oder Kaliumhydroxid oder -carbonat) in die entsprechenden Metall-, insbesondere Alkalimetall- oder Erdalkalimetall- oder in die entsprechenden Ammoniumsalze umgewandelt werden.

Bevorzugt werden die Verbindungen der Formel I, wie zuvor beschrieben oder nachfolgend als bevorzugt beschrieben eingesetzt, einschließlich der stereoisomeren Formen. Besonders bevorzugt werden die Verbindungen der Formel 1-1 eingesetzt, wobei R¹, R², R³ und R⁴ eine zuvor oder nachfolgend angegebene Bedeutung haben.

Die Verbindungen der Formel I, wie zuvor beschrieben, und/oder deren Salze, Tautomere, Stereoisomere und/oder Solvate, einschließlich deren Mischungen in allen Verhältnissen können die Haut bräunen. Glucuronolactonacetonid, die Verbindung nach Formel Ia, wie nachfolgend beschrieben, zeigt bei identischer Konzentration in der Testformulierung einen Farbeffekt vergleichbar mit Erythrulose.

Bei einer Kombination mindestens einer der Verbindungen nach Formel I, wie zuvor beschrieben, mit Dihydroxyaceton zur Verwendung in kosmetischen Formulierungen, die dem Färben der Haut dienen, ist eine bevorzugte Rotverschiebung des erzielten Farbtons zu beobachten.

Somit kann mit Dihydroxyaceton oder eine Mischung von Selbstbräunungssubstanzen enthaltend Dihydroxyaceton als Selbstbräuner und bei Verwendung von zumindest einer Verbindung nach Formel I, wie zuvor beschrieben, die Selbstbräunung der Haut mit einer natürlicher aussehenden Färbung durchgeführt werden, ohne einen unerwünschten Gelbstich der gefärbten Haut.

Im gesamten Dokument wird der Begriff Selbstbräuner bzw. Selbstbräunungssubstanz bzw. Selbstbräunersubstanz synonym verwendet. Mit diesen Begriffen wird eine Substanz bezeichnet, die die Haut färbt.

Das Prinzip der Färbung unter Ausbildung von Melanoiden ist das grundlegende Färbeprinzip der Selbstbräunungssubstanzen, die im Sinne einer Maillard-Reaktion oder über eine Michael-Addition reagieren. Obwohl die Verbindungen der Formel I nach den bisherigen Kenntnissen nicht über einen derartigen Bräunungsmechanismus verfügen, so kann das Färbevermögen solcher klassischer Maillard-Selbstbräunungssubstanzen durch Verwendung von zumindest einer Verbindung nach Formel I, wie zuvor beschrieben, dennoch überraschenderweise verstärkt werden.

Durch Verwendung von Verbindungen nach Formel I, wie zuvor beschrieben oder als bevorzugt beschrieben, kann der Färbeprozess mit Dihydroxyaceton verstärkt und/oder der erzielte Farbton verbessert werden. Demzufolge versteht man unter einem Bräunungsverstärker eine Verbindung nach Formel I, die in der Lage ist, beim Färben der Haut mit Dihydroxyaceton einen gegebenenfalls dunkleren, Farbton zu erzielen, der mehr nach Rot verschoben ist, als ein Farbton, der mit Dihydroxyaceton oder einer Mischung von Selbstbräunungssubstanzen enthaltend Dihydroxyaceton allein, erzielt wird.

Außerdem können Verbindungen nach Formel I, wie zuvor beschrieben oder bevorzugt beschrieben, einen Kontrastreduktionseffekt aufweisen, der in Anwendung mit Dihydroxyaceton oder einer Mischung von Selbstbräunungssubstanzen enthaltend Dihydroxyaceton, eine ungleichmäßige Hautfärbung reduziert und somit den Kontrast zwischen stärker und weniger stark gefärbten Hautpartien herabsetzt. Dabei kann eine derartige ungleichmäßige Hautfärbung durch eine ungleichmäßige Pigmentierung und/oder eine unterschiedliche Verteilung der Hornhaut zustande kommen. Ein Kontrastreduktionsmittel ist demzufolge eine Substanz, die eine ungleichmäßige Hautfärbung reduziert, in dem sie den Kontrast zwischen stärker und weniger stark gefärbten Hautpartien herabsetzt.

Ein weiterer Gegenstand der Erfindung ist demzufolge die Verwendung mindestens einer Verbindung der Formel I, wie zuvor beschrieben, und/oder deren Salze, Tautomere, Stereoisomere und/oder Solvate, einschließlich deren Mischungen in allen Verhältnissen als Kontrastreduktionsmittel.

In Kombination mit Dihydroxyaceton gelingt es, eine Färbung der Haut mit natürlicheren, insbesondere in den roten Farbbereich verschobenen, Hautfärbungen zu erzielen, wobei zusätzlich eine vorteilhafte Kontrastreduzierung ungleichmäßig gefärbter Hautpartien möglich ist. Zudem kann bei Auftragung auf die Haut die Hautaustrocknung ebenfalls vorteilhaft durch Verbindungen nach Formel I, wie zuvor beschrieben, reduziert werden.

Eine Kontrastreduktion kann daher insbesondere durch Zubereitungen erzielt werden, in denen erfindungsgemäße Kombinationen aus Dihydroxyaceton oder einer Mischung von Selbstbräunungssubstanzen enthaltend Dihydroxyaceton und zumindest einer Verbindung nach Formel I, wie zuvor beschrieben, zusätzlich mit einem die biochemische Melaninbildung hemmenden Stoff zusammengebracht werden. Die Vereinigung von bräunenden Mischungen, mit melanogenesehemmenden Substanzen bewirkt, dass bereits hyperpigmentierierte Hautareale ihre hohen Melaninkonzentrationen verlieren und sich der durch das Färbemittel an der Hautoberfläche erzeugte Farbton großflächig durchsetzt.
Zur Kombination eignen sich marktübliche Melanogeneseinhibitoren wie z.B. Ascorbinsäure und deren Derivate, Niacinamid, Emblica, Ellagsäure, Maulbeerbaumextrakt, Kojisäure, Süßholzwurzelextrakt, Rucinol, Hydrochinon, Azelainsäure, Arbutin oder Magnesium-ascorbyl-phosphat.

Es wurde weiterhin überraschenderweise gefunden, dass die Verbindungen der Formel I, wie nachfolgend beschrieben, sich sehr gut als direktziehende Farbstoffe für die Färbung von keratinhaltigen Fasern eignen.

Ein Gegenstand der Erfindung ist daher ebenfalls die Verwendung mindestens einer Verbindung der Formel I 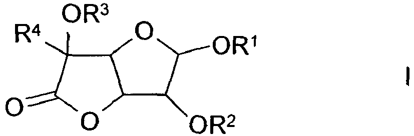
wobei R¹ und R² jeweils unabhängig voneinander Aryl,
eine geradkettige oder verzweigte Alkylgruppe mit 1 bis 30 C-Atomen, die mit Aryl substituiert sein kann oder
eine geradkettige oder verzweigte Alkenylgruppe mit 2 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
   d) durch mindestens eine geradkettige oder verzweigte Alkylgruppe mit 1 bis 30 C-Atomen und/oder
   e) durch mindestens eine Arylgruppe mit 6 bis 12 C-Atomen und/oder
   f) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
   Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
   Alkyl eine geradkettige oder verzweigte Alkylgruppe mit 1 bis 30 C-Atomen bedeutet,
   und/oder deren Salze, Tautomere, Stereoisomere und/oder Solvate, einschließlich deren Mischungen in allen Verhältnissen zur Färbung von keratinhaltigen Fasern.

Ein weiterer Gegenstand der Erfindung ist die Verwendung der Verbindungen der Formel I, wie zuvor beschrieben, und/oder deren Salze, Tautomere, Stereoisomere und/oder Solvate, einschließlich deren Mischungen in allen Verhältnissen, als direktziehender Farbstoff zur Herstellung eines Mittels zum Färben von keratinhaltigen Fasern.

Unter keratinhaltigen Fasern sind vorzugsweise menschliche Haare, Wolle, Pelze oder Federn zu verstehen. Die erfindungsgemäßen Verbindungen eignen sich prinzipiell aber auch zum Färben anderer Naturfasern, wie beispielsweise Baumwolle, Jute, Sisal, Leinen oder Seide, oder zum Färben modifizierter Naturfasern, wie beispielsweise Regeneratcellulose, Nitro-, Alkyl- oder Hydroxyalkyl- oder Acetylcellulose. Besonders bevorzugt ist die keratinhaltige Faser menschliches Haar.

Der erfindungsgemäß verwendete Begriff "Färben von keratinhaltigen Fasern" umfasst jedwede Form der Farbveränderung der Fasern. Umfasst sind insbesondere die unter den Begriffen Tönung, Blondierung, oxidativer Färbung, semipermanenter Färbung, permanenter Färbung sowie temporärer Färbung umfassten Farbveränderungen. Ebenfalls können Farbveränderungen auftreten, die ein helleres Farbergebnis im Vergleich zur Ausgangsfarbe aufweisen, wie beispielsweise Blondierungen. Unter dem Begriff "Färben von keratinhaltigen Fasern" wird bevorzugt eine Tönung oder eine temporäre, semipermanente oder permanente Färbung verstanden.

Eine geradkettige oder verzweigte Alkylgruppe mit 1 bis 10 C-Atomen ist beispielsweise Methyl, Ethyl, *iso*-Propyl, Propyl, Butyl, sec-Butyl oder tert-Butyl, Pentyl, 1-, 2- or 3-Methylbutyl, 1,1-, 1,2- or 2,2-Dimethylpropyl, 1-Ethylpropyl, n-Hexyl, n-Heptyl, n-Octyl, n-Nonyl oder n-Decyl.

Eine geradkettige oder verzweigte Alkylgruppe mit 1 bis 30 C-Atomen umfasst die zuvor beschriebene Gruppe der geradkettigen oder verzweigten Alkylgruppe mit 1 bis 10 C-Atomen und Undecanyl, Dodecanyl, Tridecanyl, Tetradecanyl, Pentadecanyl, Hexadecanyl, Heptadecanyl, Oktadecanyl, Nonadecanyl, Eicosanyl, Heneicosanyl, Docosanyl, Tricosanyl, Tetracosanyl, Pentacosanyl, Hexacosanyl, Heptacosanyl, Octacosanyl, Nonacosanyl und Triacontanyl.

Der vorliegend verwendete Terminus "Alkyl" bedeutet eine geradkettige oder verzweigte Alkylgruppe mit 1 bis 30 C-Atomen, vorzugsweise eine Alkylgruppe mit 1 bis 10 C-Atomen.

Ein geradkettiges oder verzweigtes Alkenyl mit 2 bis 30 C-Atomen, wobei auch mehrere Doppelbindungen vorhanden sein können, ist beispielsweise Ethenyl, Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis-C₃₀H₄₉; vorzugsweise Ethenyl, Allyl, 2- oder 3-Butenyl, Isobutenyl, sek.-Butenyl, 4-Pentenyl, iso-Pentenyl, Hexenyl oder Decenyl. Besonders bevorzugte Alkenylgruppen sind Ethenyl, 2- oder 3-Butenyl.

Der vorliegend verwendete Terminus "Aryl" bedeutet eine Arylgruppe mit 6 bis 12 C-Atomen, beispielsweise Phenyl, Naphthyl oder Phenanthryl, die durch Alkyl, OH, OAlkyl, NH₂, NH-Alkyl, N(Alkyl)₂, C(O)Alkyl, O-C(O)-Alkyl oder C(O)-OAlkyl substituiert sein kann. Bevorzugt ist die Arylgruppe unsubstituiertes oder entsprechend substituiertes Phenyl.

Der Substituent R³ bedeutet vorzugsweise eine geradkettige oder verzweigte Alkylgruppe mit 1 bis 4 C-Atomen oder H, besonders bevorzugt H.

Der Substituent R⁴ bedeutet H oder OR³, wobei R³ eine zuvor angegebene Bedeutung oder bevorzugte Bedeutung haben kann. Der Substituent R⁴ bedeutet besonders bevorzugt H.

R¹ und R² bilden vorzugsweise zusammen einen fünfgliedrigen Ring, der durch mindestens eine geradkettige oder verzweigte Alkylgruppe mit 1 bis 10 C-Atomen substituiert sein kann
und/oder durch mindestens eine geradkettige oder verzweigte Alkenylgruppe mit 1 bis 10 C-Atomen enthaltend eine oder mehrere Doppelbindungen substituiert sein kann, die durch mindestens eine Phenylgruppe substituiert sein kann, wobei die Phenylgruppe durch Alkyl, OH, OAlkyl, C(O)Alkyl, O-C(O)Alkyl und/oder C(O)-OAlkyl substituiert sein kann
und/oder durch eine Phenylgruppe substituiert sein kann, wobei die Phenylgruppe durch Alkyl, OH, OAlkyl, C(O)Alkyl, O-C(O)Alkyl und/oder C(O)-OAlkyl substituiert sein kann, wobei Alkyl eine zuvor genannte Bedeutung oder bevorzugt genannte Bedeutung hat. Bevorzugt ist der sich bildende fünfgliedrige Ring durch Methyl, Ethyl, 2-Ethylhexyl, Phenyl, Methoxyphenyl, Ethoxyphenyl, Methoxy-hydroxy-phenyl, 4-Hydroxy-3-methoxy-phenyl-ethenyl monosubsituiert oder entsprechend durch zwei Methylgruppen, zwei Phenylgruppen, eine Methylgruppe und eine Phenylgruppe oder eine Methylgruppe und eine Tetradecylgruppe disubstituiert.

Bilden die Substituenten R¹ und R² keinen Ring, so ist R² vorzugsweise H und R¹ bedeutet eine Alkylgruppe mit 1 bis 30 C-Atomen, Arylmethyl, Arylethenyl oder Aryl, wobei Aryl eine Bedeutung hat, wie zuvor beschrieben.

Bilden die Substituenten R¹ und R² keinen Ring und R² ist H, so ist R¹ ganz besonders bevorzugt Methyl, Ethyl, 2-Ethylhexyl, n-Tetradecyl, Benzyl, Phenyl oder Phenyl-ethenyl.

Bevorzugte Einzelverbindungen der Formel I sind
die Verbindungen (Ia) bis (Im) 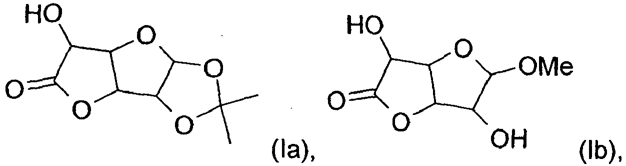 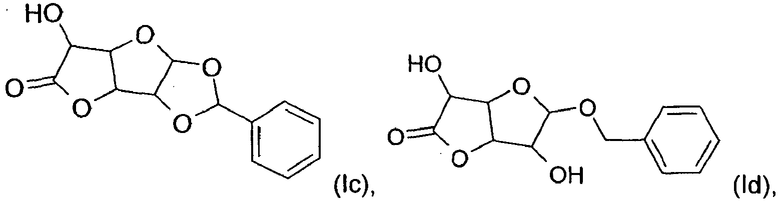 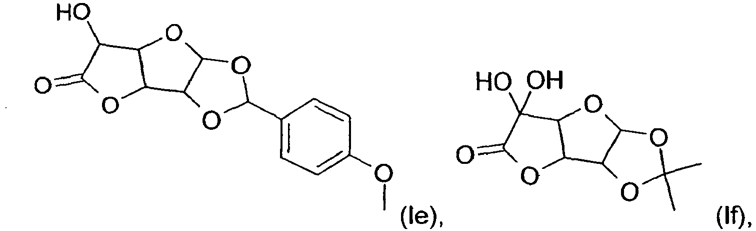 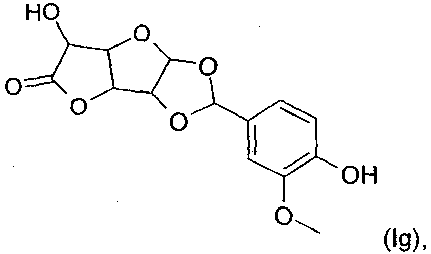 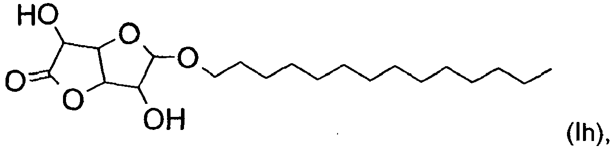 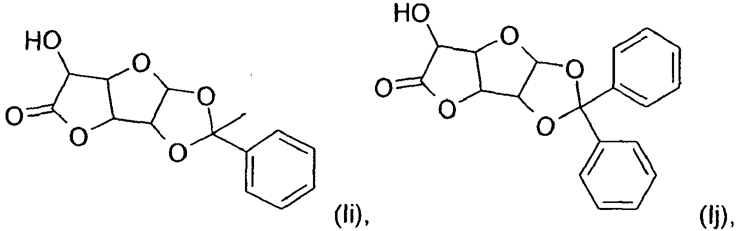 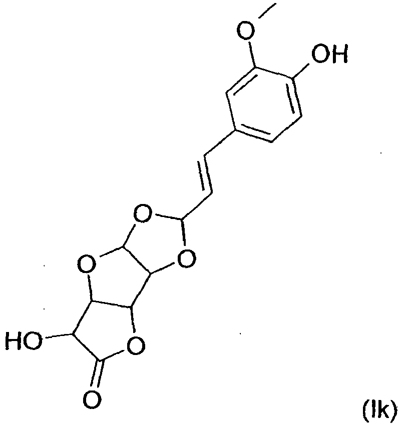 oder 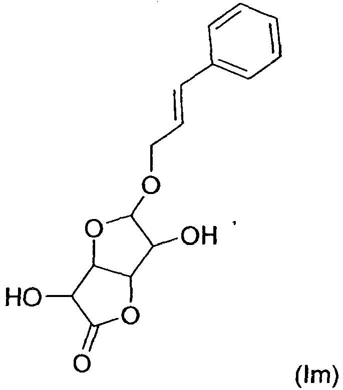 und/oder deren Salze, Tautomere, Stereoisomere und/oder Solvate, einschließlich deren Mischungen in allen Verhältnissen. Bekannte Verbindungen finden sich in z.B. FR2717176 A1.

Die Einzelverbindungen sind bevorzugt Diastereomere und können ebenfalls gemäß der Stereochemie der Formel I-1 beschrieben werden.

Selbstverständlich ist es möglich, durch die Wahl der Substitution der Arylgruppe in den Verbindungen der Formel I, die zu erzielende Farbe der keratinhaltigen Faser zu variieren. Je stärker beispielsweise die Aromatizität der entsprechenden Verbindung der Formel I, desto dunkler erwartet man das Färbeergebnis.

Besonders bevorzugt sind die Verbindungen der Formel (la), (Ib) und (Ic), ganz besonders bevorzugt sind die Verbindungen der Formel (la) oder (Ic).

Als vorteilhafte Selbstbräuner in einer Dihydroxyaceton enthaltenden Mischung oder Zubereitung können unter anderem eingesetzt werden: Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon) oder 2-Hydroxy-1,4-naphtochinon (Lawson) oder eine Mischung der genannten Verbindungen. Besonders bevorzugt wird Erythrulose in der Dihydroxyaceton enthaltenden Mischung eingesetzt.

Die mindestens eine Verbindung der Formel I, wie zuvor beschrieben oder als bevorzugt beschrieben, kann auch zusammen mit einer Mischung von Selbstbräunungssubstanzen enthaltend mindestens Dihydroxyaceton und einen weiteren Selbstbräuner, ausgewählt aus der zuvor genannten Gruppe, erfindungsgemäß verwendet werden. Beispielhaft besteht die erfindungsgemäß zu verwendende Mischung aus Dihydroxyaceton und mindestens einer weiteren Selbstbräunungssubstanz, wie zuvor beschrieben. Diese Mischung kann dann erfindungsgemäß mit mindestens einer Verbindung der Formel I kombiniert werden und in kosmetischen, dermatologischen oder pharmazeutischen Zubereitungen eingesetzt werden, wie nachfolgend beschrieben.

Ganz besonders bevorzugt wird Dihydroxyaceton ohne weitere Selbstbräunungssubstanzen der zuvor genannten Gruppe an Selbstbräunungssubstanzen eingesetzt.

Die Verbindungen der Formel I, wie zuvor beschrieben, und auch die Ausgangsstoffe zu ihrer Herstellung sind zum Teil kommerziell erhältlich oder werden nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.
In der Regel ist Glucuronolacton (R³ = H und R⁴ = H) oder ein Glucuronolacton-Derivat der Formel II oder II-1 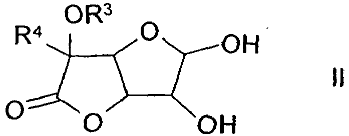 oder 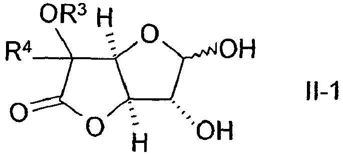 der geeignete Ausgangsstoff für die Verbindungen der Formel I oder entsprechend Formel I-1, wie zuvor beschrieben oder als bevorzugt beschrieben.
Die Verbindungen der Formel I, in denen R¹ und R² zusammen einen fünfgliedrigen Ring bilden, entstehen beispielsweise durch Lewis-säurekatalysierte Umsetzung von Glucuronolacton oder einer Verbindung der Formel II oder II-1 mit einem entsprechenden Aldehyd oder Keton.

Die Synthese von Verbindungen der Formel I, in denen R¹ und R² jeweils unabhängig voneinander Aryl oder eine eine geradkettige oder verzweigte Alkylgruppe mit 1 bis 30 C-Atomen, die mit Aryl substituiert sein kann oder eine geradkettige oder verzweigte Alkenylgruppe mit 2 bis 30 C-Atomen, enthaltend eine oder mehrere Doppelbindungen, die mit Aryl substituiert sein kann, bedeuten und wobei entweder R¹ oder R² H bedeuten kann, wie zuvor beschrieben, erfolgt durch Umsetzung von Glucuronolacton oder einer Verbindung der Formel II oder der Formel II-1 mit einem entsprechenden Alkohol. Die Umsetzung findet in der Regel unter Säurekatalyse statt. Die genauen Reaktionsbedingungen dieser Veretherungen sind dem Synthesefachmann hinlänglich bekannt. Die Kinetik der beiden OH-Gruppen im Glucuronolacton oder der Verbindung der Formel II oder der Formel II-1 ist verschieden, so dass die Veretherung entsprechend durch Zudosierung und Schutzgruppenchemie gesteuert werden kann.

Weitere Details sind in den Beispielen aufgeführt, die entsprechend auch für die allgemeine Synthesebeschreibung gelten.

Ein weiterer Gegenstand der Erfindung sind Zubereitungen, enthaltend Dihydroxyaceton (DHA) und zumindest eine Verbindung nach Formel I, wie zuvor beschrieben oder als bevorzugt beschrieben.

Bevorzugt enthält die Zubereitung zumindest Dihydroxyaceton in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung.

Bevorzugt enthält die Zubereitung die zumindest eine Verbindung der Formel I, wie zuvor beschrieben oder als bevorzugt beschrieben, in einer Menge von 0,01 bis 20 Gew.-%, besonders bevorzugt in einer Menge von 0,5 bis 15 Gew.-% und ganz besonders bevorzugt in einer Menge von 1 bis 8 Gew.-%, bezogen auf die Gesamtmenge der Zubereitung.

Die Zubereitung, wie zuvor beschrieben, enthält in der Regel einen für kosmetische, pharmazeutische oder dermatologische Zubereitungen geeigneten Träger, wie nachfolgend noch beschrieben.

Die Zubereitungen können in bevorzugter Weise Hilfsstoffe enthalten, wie beispielsweise kosmetische Öle (z.B. Caprylic/Capric Triglycerides, C12-15 Alkyl Benzoate, Isopropylmyristat, Arylalkyl Benzoate wie z.B. Phenethylbenzoat (X-Tend 226) oder Ölkomponenten der Marke Cosmacol wie Dimyristyl Tartrate, Tri C14-C15 Alkyl Citrate, C12-C13 Alkyl Lactate, Tridecyl Salicylate, C12-C13 Alkyl Octanoate, C12-C13 Alkyl Malate, C12-C13 Alkyl Citrate, C12-C13 Alkyl Tartrate), oder polarprotische Hilfsstoffe (z.B. Propylenglycol, Glycerin, Isopropanol, Ethanol) oder sog. Lösungsvermittler (z.B. Butylphthalimide, Isopropylphthalimide, Dimethylisosorbide). Ganz besonders bevorzugte kosmetische Öle sind C12-C13 Alkyl Lactate, kommerziell erhältlich als Cosmacol ELI und Phenethylbenzoat, kommerziell erhältlich als X-Tend 226.

Zubereitungen mit Selbstbräunereigenschaften, insbesondere solche, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Daher kann die erfindungsgemäße Zubereitung enthaltend mindestens eine Verbindung der Formel I, wie zuvor mit den angegebenen und auch bevorzugt genannten Substituenten beschrieben sowie den Einzelverbindungen und mindestens einen Selbstbräuner, vorzugsweise auch Formaldehydfänger sowie gegebenenfalls Flavonoide zur Verbesserung des Geruches enthalten.

Die für erfindungsgemäße Zubereitungen beanspruchten Verbindungen der Formel I, sowie die entsprechenden bevorzugten Verbindungen, können aber auch ihrerseits zur Geruchsverbesserung beitragen, wie im Beispielteil beschrieben. Insbesondere zeichnen sich die Verbindungen der Formel I, wie zuvor beschrieben, dadurch aus, dass sie bei Lagerung in einer Zubereitung keine Fehlgerüche erzeugen.

Vorzugsweise wird der Formaldehydfänger ausgewählt aus der Gruppe Alkalimetall-, Erdalkalimetall- oder Ammoniumdisulfit. Besonders bevorzugt ist eine Zubereitung, die in Kombination DHA Plus, eine Mischung aus DHA, Natriumdisulfit und Magnesiumstearat, enthält.

DHA Plus ist eine Produktmischung, welche zur Maskierung, Eliminierung oder Neutralisierung des Formaldehyds Natriummetabisulfit, gleichbedeutend mit Na₂S₂O₅ oder INCI: sodium disulfite, enthält. Der Zusatz von Natriummetabisulfit in fertigen Formulierungen führt zur signifikanten Reduktion oder Unterbindung des unangenehmen Geruchs. DHA Plus wird von der Firma Merck, Darmstadt vertrieben.

Die erfindungsgemäße Zubereitung enthaltend mindestens eine Verbindung der Formel I, wie zuvor mit den angegebenen und auch bevorzugt genannten Substituenten beschrieben sowie den genannten Verbindungen, und mindestens Dihydroxyaceton als Selbstbräuner, kann besonders bevorzugt Flavonoide zur Verbesserung des Geruches und gegebenenfalls zur Bräunungsbeschleinigung enthalten.

Das Flavonoid wirkt dabei zusätzlich als Stabilisator für den Selbstbräuner bzw. die Selbstsbräunungssubstanzen und/oder reduziert oder vermeidet oder verbessert lagerabhängige Fehlgerüche, die auch durch enthaltene Zusatz- oder Hilfsstoffe entstehen können.

Vorzugsweise handelt es sich um ein Flavonoid, bei dem eine oder mehrere phenolische Hydroxygruppen durch Veretherung oder Veresterung blockiert sind. Beispielsweise haben sich Hydroxyethyl-substituierte Flavonoide, wie vorzugsweise Troxerutin, Troxequercetin, Troxeisoquercetin oder Troxeluteolin, und Flavonoid-sulfate oder Flavonoid-phosphate, wie vorzugsweise Rutinsulfate, dabei als besonderes gut geeignete Flavonoide erwiesen. Besonders bevorzugt im Sinne der erfindungsgemäßen Verwendung sind Rutinsulfat und Troxerutin. Ganz besonders bevorzugt ist die Verwendung von Troxerutin.

Die bevorzugten Flavonoide verfügen über einen nicht positiv geladenen Flavangruhdkörper. Es wird vermutet, dass durch diese Flavonoide Metallionen wie z.B. Fe²⁺/Cu²⁺ komplexiert werden und so Autooxidationsvorgänge bei Duftstoffen oder Verbindungen, deren Abbau zu Fehlgerüchen führt, verhindert bzw. vermindert werden.

Besonders bevorzugt ist eine Zubereitung, die neben mindestens einer Verbindung der Formel I DHA Rapid enthält. DHA Rapid ist eine Produktmischung enthaltend Dihydroxyaceton und Troxerutin, der Firma Merck, Darmstadt. Diese besonders bevorzugte Zubereitung kann gegebenenfalls auch noch einen Formaldehydfänger, beispielsweise Natriumdisulfit enthalten.

Entsprechende Vormischungen und Zubereitungen, die Formaldehydfänger sowie gegebenenfalls Flavonoide zur Verbesserung des Geruches auf der Haut enthalten, sind in der Deutschen Patentanmeldung DE 10 2007 013 368 A1 beschrieben, deren diesbezüglicher Inhalt ausdrücklich auch zum Offenbarungsgehalt der vorliegenden Anmeldung gehört.

Bei den Zubereitungen handelt es sich dabei üblicherweise um topisch anwendbare Zubereitungen, beispielsweise kosmetische oder dermatologische Formulierungen oder Medizinprodukte. Die Zubereitungen enthalten in diesem Fall einen kosmetisch oder dermatologisch geeigneten Träger und je nach gewünschtem Eigenschaftsprofil optional weitere geeignete Inhaltsstoffe. Handelt es sich um pharmazeutische Zubereitungen, so enthalten die Zubereitungen in diesem Fall einen pharmazeutisch verträglichen Träger und optional weitere pharmazeutische Wirkstoffe.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Im Sinne der vorliegenden Erfindung wird neben dem Begriff Zubereitung gleichbedeutend auch der Begriff Mittel oder Formulierung verwendet.

Die Zubereitungen können die genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden.

Weitere bevorzugte Kombinationen von Ausführungsformen sind in den Ansprüchen offenbart.

Ein Gegenstand der Erfindung ist auch ein Verfahren zur Herstellung einer Zubereitung, wie zuvor beschrieben, wobei mindestens eine Verbindung nach Formel I, wie zuvor beschrieben oder als bevorzugt beschrieben, mit einem Träger und gegebenenfalls mit weiteren Aktiv- oder Hilfsstoffen vermischt wird. Dann wird ggf. zumindest eine weitere Selbstbräunersubstanz zugegeben und vermischt und als letztes Dihydroxyaceton zugegeben und gemischt. Geeignete Trägerstoffe sowie Aktiv- oder Hilfsstoffe sind im nachfolgenden Teil ausführlich beschrieben.

In den beschriebenen Zubereitungen, die erfindungsgemäß mindestens eine Verbindung nach Formel I, wie zuvor beschrieben oder als bevorzugt beschrieben, und Dihydroxyaceton enthalten, können weiterhin auch Farbpigmente enthalten sein, wobei der Schichtaufbau der Pigmente nicht limitiert ist.

Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Bevorzugte Zubereitungen können neben den Verbindungen nach Formel I, mindestens Dihydroxyaceton als Selbstbräunungssubstanz sowie den gegebenenfalls anderen Inhaltsstoffen weitere organische UV-Filter enthalten, sogenannte hydrophile oder lipophile Sonnenschutzfilter, die im UVA-Bereich und/oder UVB-Bereich und(/oder IR und/oder VIS-Bereich (Absorber) wirksam sind. Diese Substanzen können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben.

Geeignete organischen UV-schützende Substanzen sind bevorzugt aus der folgenden Liste auszuwählen: Ethylhexyl salicylate, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Die Zubereitungen können neben den Verbindungen der Formel I, mindestens Dihydroxyaceton als Selbstbräunungssubstanz sowie den gegebenenfalls anderen organischen UV-Filtern, wie zuvor beschrieben, weitere anorganische UV-Filter, sogenannte partikuläre UV-Filter enthalten.

Diese Kombinationen mit partikulären UV-Filtern sind sowohl als Pulver als auch als Dispersion oder Paste möglich.
Hierbei sind sowohl solche aus der Gruppe der Titandioxide wie z.B. gecoatetes Titandioxid (z.B. Eusolex^{®} T-2000, Eusolex^{®}T-AQUA, Eusolex^{®}T-AVO, Eusolex^{®}T-OLEO), Zinkoxide (z.B. Sachtotec^{®}), Eisenoxide oder auch Ceroxide und/oder Zirkonoxide bevorzugt.

Weiter kann es bevorzugt sein, wenn die Zubereitungen anorganische UV-Filter enthalten, die mit üblichen Methoden, wie beispielsweise in Cosmetics & Toiletries, 1990, 105, 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Diese anorganischen UV-Filter werden in der Regel in einer Menge von 0,1 Gewichtsprozent bis 25 Gewichtsprozent, vorzugsweise 2 Gew.-% - 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen.

Bevorzugte Zubereitungen können auch mindestens einen weiteren kosmetischen Wirkstoff enthalten, beispielsweise ausgewählt aus Antioxidantien, Anti-Aging-Wirkstoffen, Anti-Falten, Anti-Schuppen, Anti-Akne, deodorants, Anti-Cellulite Wirkstoffen, hautaufhellenden Wirkstoffen oder Vitaminen.

Geeignete anti-aging Wirkstoffe, insbesondere für hautpflegende Zubereitungen, sind vorzugsweise sogenannte kompatible Solute. Vorzugsweise werden als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), ß-Mannosylglycerat (Firoin), ß- Mannosylglyceramid (Firoin-A) oder/und Dimannosyl-di-inositolphosphat (DMIP) oder ein optisches Isomer, Derivat, z.B. eine Säure, ein Salz oder Ester dieser Verbindungen oder Kombinationen davon eingesetzt.

Additional können als anti-aging Wirkstoffe Produkte der Firma Merck wie z.B. 5,7-Dihydroxy-2-methyl-chromon, vermarktet unter dem Handelsnamen RonaCare®Luremine, oder die Handelsprodukte RonaCare®Isoquercetin, RonaCare®Tilirosid oderRonaCare®Cyclopeptide 5 verwendet werden.

Die Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Beispiele von Verbindungen mit hautaufhellender Aktivität sind Hydrochinon, Kojisäure, Arbutin, Aloesin, Niacinamide, azelainsäure, Elaginsäure, Maulbeerbaumextract, Magnesium-ascorbyl-phosphat, Süßholzwurzelextrakt, Emblica, Ascorbinsäure oder Rucinol.

Die einzusetzenden Zubereitungen können als weitere Inhaltsstoffe Vitamine enthalten.

Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂), insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den flavonoidhaltigen Vormischungen oder Zubereitungen üblicherweise bei kosmetischer Anwendung in Bereichen von 0,01 bis 5,0 Gew.-%, bezogen auf das Gesamtgewicht, zugesetzt.

Die beschriebenen Retinoide sind gleichzeitig auch wirksame Anti-Cellulite-Wirkstoffe. Ein ebenfalls bekannter Anti-Cellulite-Wirkstoff ist Koffein.

Die genannten Bestandteile der Zubereitung können in der üblichen Weise eingearbeitet werden, mit Hilfe von Techniken, die dem Fachmann wohl bekannt sind.

Geeignet sind Zubereitungen für eine äußerliche Anwendung, beispielsweise als Creme oder Milch (O/W, W/O, O/W/O, W/O/W), als Lotion oder Emulsion, in Form ölig-alkoholischer, ölig-wässriger oder wässrigalkoholischer Gele bzw. Lösungen auf die Haut aufgesprüht werden kann. Sie können als feste Stifte vorliegen oder als Aerosol konfektioniert sein.

Als Anwendungsform der einzusetzenden Zubereitungen seien bevorzugt genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole Pflaster, Umschläge, Verbände und Sprays.

Bevorzugte Hilfsstoffe stammen aus der Gruppe der Konservierungsstoffe, Stabilisatoren, Lösungsvermittler, Färbemittel, Geruchsverbesserer.

Salben, Pasten, Cremes und Gele können die üblichen Trägerstoffe enthalten, die für die topische Verabreichung geeignet sind, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Ein bevorzugter Lösungsvermittler generell ist 2-Isopropyl-5-methyl-cyclohexancarbonyl-D-Alaninmethylester.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe,enthalten.

Weitere typische kosmetische Anwendungsformen sind auch Lippenstifte, Lippenpflegestifte, Puder-, Emulsions- und Wachs-Make up sowie Sonnenschutz-, Prä-Sun- und After-Sun-Präparate.

Zu den bevorzugten Zubereitungsformen gehören insbesondere auch Emulsionen.

Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen.

Die wässrige Phase der einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, und andere in der Kosmetik gewöhnlich verwendete Ingredienzien.

Die Verbindungen der Formel I, wie zuvor beschrieben oder als bevorzugt beschrieben, werden insbesondere in Mitteln zum Färben der keratinhaltigen Fasern, insbesondere zum Färben menschlicher Haare, eingesetzt, die beispielsweise ausgewählt werden aus einem Farbfestiger, einer Farbfönlotion, einem Farbfönschaum, einer Farbspülung, einem Farbgel oder einer Farbcreme. Sie können jedoch auch in Mitteln zur permanenten Haarfärbung, beispielsweise in Mehrkomponentensystemen enthalten sein.

Die entsprechenden Mittel zum Färben der keratinhaltigen Fasern, wie zuvor beschrieben, enthalten die Verbindung(en) der Formel I vorzugsweise in Mengen oberhalb von 0,01 Gew.-% und unterhalb von 10 Gew.-%, jeweils bezogen auf das gesamte Mittel. Bevorzugte Mittel zum Färben keratinhaltiger Fasern sind dadurch gekennzeichnet, dass sie die Verbindung(en) der Formel I in Mengen von 0,05 bis 5 Gew.-%, vorzugsweise von 0,1 bis 2,5 Gew.-%, besonders bevorzugt von 0,25 bis 1,5 Gew.-% und insbesondere von 0,4 bis 1 Gew.-%, jeweils bezogen auf das gesamte Mittel enthalten.

Die entsprechenden Mittel enthaltend mindestens eine Verbindung der Formel I dienen der Farbveränderung keratinhaltiger Fasern, wie zuvor beschrieben, insbesondere menschlicher Haare. Die Farbveränderung kann allein aufgrund der Verbindung(en) der Formel I erfolgen, die Mittel können aber auch zusätzlich weitere farbverändernde Substanzen enthalten, beispielsweise weitere direktziehende Farbstoffe und/oder Oxidationsfärbemittel. Die mindestens eine Verbindung der Formel I wird bevorzugt in Färbemitteln verwendet, die zusätzlich 0,001 bis 5 Gew.-% eines oder mehrerer Oxidationsfarbstoffvorprodukte und/oder direktziehender Farbstoffe enthalten.

Oxidationsfarbstoffe entstehen durch oxidative Kupplung einer oder mehrerer Entwicklungskomponenten untereinander oder mit einer oder mehreren Kupplerkomponenten. Kuppler- und Entwicklerkomponenten werden auch als Oxidationsfarbstoffvorprodukte bezeichnet.

Das Mittel zum Färben der keratinhaltigen Fasern enthaltend mindestens eine Verbindung der Formel I, wie zuvor beschrieben, kann als Einkomponentenmittel, als Zweikomponentenmittel oder als Dreikomponentenmittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind. Das einzusetzende Mittel wird bei solchen Systemen vom Verbraucher direkt vor der Anwendung durch Vermischen der Komponenten hergestellt.

Beispielsweise wird bei der permanenten Haarfärbung oft ein Mittel enthaltend das Oxidationsmittel als erste Komponente getrennt von dem weiteren Färbemittel enthaltend beispielsweise die Oxidationsfarbstoffvorprodukte verwendet.

Ein weiterer Gegenstand der Erfindung ist ein Verfahren zum Färben von keratinhaltigen Fasern, worin ein Mittel zum Färben von keratinhaltigen Fasern enthaltend mindestens eine Verbindung der Formel I, wie zuvor beschrieben oder als bevorzugt beschrieben, mindestens einmal täglich oder mindestens zwei Mal oder mehrere Male hintereinander auf die keratinhaltige Faser aufgebracht, einige Zeit, üblicherweise ca. 20 bis 45 Minuten, auf der Faser belassen, und anschließend wieder ausgespült oder mit einem Shampoo ausgewaschen wird.

Das derartig beschriebene erfindungsgemäße Verfahren zum Färben von keratinhaltigen Fasern ist sehr mild, da auf alkalisierende Vorbehandlungsmittel verzichtet werden kann.

Es ist jedoch auch möglich, eine Vorbehandlung der keratinhaltigen Fasern durchzuführen und dann das Mittel enthaltend die mindestens eine Verbindung der Formel I aufzutragen.

Ein solches Vorbehandlungsmittel kann basisch, sauer oder neutral sein, ist jedoch bevorzugt basisch. Bevorzugt weist das Vorbehandlungsmittel NH₃ und/oder (NH₄)CI₃ auf. Üblicherweise wird der Vorbehandlungsschritt vor dem Färbeschritt durchgeführt, es ist aber auch eine gleichzeitige Durchführung von Vorbehandlungsschritt und Färbeschritt bei dementsprechender Formulierung denkbar.

Die entsprechenden Mittel zum Färben enthaltend mindestens eine Verbindung der Formel I werden hergestellt, in dem zumindest ein Verbindung nach Formel I, wie zuvor beschrieben, mit zumindest einem für kosmetische, dermatologische Zubereitungen geeigneten Träger und gegebenenfalls Hilfsstoffen und/oder Füllstoffen gemischt, insbesondere dispergiert und/oder emulgiert und/oder gelöst wird.

Des Weiteren können, um z.B. weitere Farbanpassungen vornehmen zu können, die Mittel enthaltend die mindestens eine Verbindung der Formel I und/oder II weitere Oxidationsfarbstoffkomponenten enthalten.

Kupplerkomponenten erlauben in der Regel mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation. Wenn die zyklische Verbindung ein Sechsring ist, so befinden sich die besagten Gruppen bevorzugt in ortho-Position oder meta-Position zueinander.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem MolVerhältnis von 1:0,5 bis 1:3, insbesondere 1:1 bis 1:2, stehen können.

Geeignete Oxidationsfarbstoffkomponenten vom Entwicklertyp sind p-Phenylendiamin und dessen Derivate. Geeignete p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-pheny(endiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Weitere geeignete p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie den physiologisch verträglichen Salzen dieser Verbindungen.

Als weitere geeignete Entwicklerkomponenten können Verbindungen eingesetzt werden, die mindestens zwei aromatische Kerne enthalten, die mit Amino- und/oder Hydroxylgruppen substituiert sind. Weitere geeignete Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Weitere geeignete zweikernige Entwicklerkomponenten werden ausgewählt aus N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es möglich sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Weitere geeignete Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo[1,5-a]pyrimidine geeignet, wobei bevorzugte Pyrazolo[1,5-a]pyrimidine ausgewählt sind aus Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)ethanol, 2-[(3-Aminopyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen. Weitere geeignete Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenyfendiamin, N,N-Bis-(2-hydroxyethyl)-p-phenylendiamin, 2-Methoxymethyl-p-phenylendiamin, N-(4-Amino-3-methylphenyl)-N-[3-(1 H-imidazol-1-yl)propyl]amin, N,M-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Weitere geeignete Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,0001 bis 10 Gew.-%, vorzugsweise 0,001 bis 5 Gew.-%, jeweils bezogen auf das gesamte Färbemittel, verwendet.

Geeignete Oxidationsfarbstoffkomponenten vom Kupplertyp werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydrochinoxalin, und/oder Gemischen aus zwei oder mehreren Verbindungen aus einer oder mehreren dieser Klassen.

Weitere verwendbare Kupplerkomponenten, wie m-Aminophenole bzw. deren Derivate, werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2 -methylphenol , 5-(2'-Hydroxyethyl)amino-2-methylphenol, 3-Diethylaminophenol, N-Cyclopentyl-3-aminophenol, 1,3-Dihydroxy-5-(methylamitio)benzol, 3-Ethylamino-4-methylphenol, 2,4-Dichlor-3-aminophenol und deren physiologisch verträglichen Salzen.

Weitere verwendbare Kupplerkomponenten, wie z.B. 3-Diaminobenzole bzw. deren Derivate, werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen.

Weitere verwendbare Kupplerkomponenten, wie z.B. o-Diaminobenzole bzw. deren Derivate, werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Weitere verwendbare Kupplerkomponenten, wie z.B. Di- beziehungsweise Trihydroxybenzole und deren Derivate, werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Weitere verwendbare Kupplerkomponenten, wie z.B. Pyridinderivate, werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methy(pyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Als Kupplerkomponente geeignete Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Als Kupplerkomponente geeignete Indolderivate werden ausgewählt aus 4-Hydroxyindol, 6-Hydroxyindol und 7-Hydroxyindol und deren physiologisch verträglichen Salzen.

Als Kupplerkomponente geeignete Indolinderivate werden bevorzugt ausgewählt aus 4-Hydroxyindolin, 6-Hydroxyindolin und 7-Hydroxyindolin und deren physiologisch verträglichen Salzen.

Als Kupplerkomponente geeignete Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Geeignete Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Amino-phenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethylamino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,0001 bis 10 Gew.-%, vorzugsweise 0,001 bis 5 Gew.-%, jeweils bezogen auf das gesamte Mittel, verwendet.

Für temporäre Färbungen werden üblicherweise Färbe- oder Tönungsmittel verwendet, die als färbende Komponente so genannte Direktzieher enthalten. Hierbei handelt es sich um Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Weiterhin können die erfindungsgemäßen Mittel mindestens einen weiteren direktziehenden Farbstoff enthalten. Dabei handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Zubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den internationalen Bezeichnungen (INCI) bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Als nichtionische direktziehende Farbstoffe eignen sich insbesondere nichtionische Nitro- und Chinonfarbstoffe und neutrale Azofarbstoffe.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die Mittel enthalten mit besonderem Vorzug zusätzlich Wasserstoffperoxid. Derartige Mittel zum Färben und gegebenenfalls gleichzeitigem Aufhellen keratinhaltiger Fasern sind besonders bevorzugt, die 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthalten.

Das Wasserstoffperoxid kann auch in Form dessen Anlagerungsverbindungen an feste Träger eingesetzt werden, bevorzugt wird Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon nH₂O₂ (n ist eine positive ganze Zahl größer 0), Harnstoffperoxid und Melaminperoxid, eingesetzt.

Ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Für eine Farbveränderung mittels Aufhellung bzw. Bleichung des Substrats, beispielsweise der Haare, wird bevorzugt in kosmetischen Mitteln neben den Oxidationsmitteln zusätzlich mindestens ein Bleichverstärker eingesetzt.

Bleichverstärker werden bevorzugt zur Steigerung der Bleichwirkung des Oxidationsmittels, insbesondere des Wasserstoffperoxids, eingesetzt. Geeignete Bleichverstärker sind

(BV-i) Verbindungen, die unter Perhydrolysebedingungen aliphatische Peroxocarbonsäuren und/oder gegebenenfalls substituierte
Perbenzoesäure ergeben, und/oder
(BV-ii) Carbonatsalze und/oder Hydrogencarbonatsalze, und/oder
(BV-iii) organische Carbonate, und/oder
(BV-iv) Carbonsäuren, und/oder
(BV-v) Peroxoverbindungen.

Bleichverstärker sind bevorzugt Peroxoverbindungen, insbesondere anorganische Peroxoverbindungen. Unter die bleichverstärkenden Peroxoverbindungen fallen keine Anlagerungsprodukte von Wasserstoffperoxid an andere Komponenten und auch nicht Wasserstoffperoxid selbst. Die Auswahl der Peroxoverbindungen unterliegt darüber hinaus keinen Beschränkungen. Bevorzugte Peroxoverbindungen sind Peroxidisulfatsalze, Persulfatsalze, Peroxidiphosphatsalze (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die Peroxidisulfate, insbesondere Ammoniumperoxidisulfat, bevorzugt. Hier sind Mittel zum Färben und gegebenenfalls gleichzeitigem Aufhellen keratinischer Fasern bevorzugt, die zusätzlich 0,01 bis 2 Gew.-% mindestens einer festen Peroxoverbindung, die ausgewählt ist aus Ammonium-, Alkalimetall- und Erdalkalimetallpersulfaten, - peroxomonosulfaten und -peroxidisulfaten enthalten, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei bevorzugte Mittel mindestens zwei verschiedene Peroxidisulfate enthalten.

Weiterhin besonders bevorzugt sind Persulfate, insbesondere das als Carosche Salz bezeichnete Gemisch aus Kaliumperoxosulfat, Kaliumhydrogensulfat und Kaliumsulfat.

Die Bleichverstärker sind in den erfindungsgemäßen, kosmetischen Mitteln bevorzugt in Mengen von 5 bis 30 Gew.-%, insbesondere in Mengen von 8 bis 20 Gew.-%, jeweils bezogen auf das Gewicht des anwendungsbereiten Mittels, enthalten.

Weiterhin hat es sich als vorteilhaft erwiesen, wenn die Färbe- und/oder Aufhellmittel nichtionogene grenzflächenaktive Stoffe enthalten.

Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen. Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Färbe- und/oder Aufhellmittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z.B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchesäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genusssäuren und Basen,
- Wirkstoffe wie Panthenol, Pantothensäure, Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyalkylether,
- Fette und Wachse wie Walrat, Bienenwachs, Montanwachs und Paraffine, Fettalkohole und Fettsäureester,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel zum Färben keratinhaltiger Fasern können die Inhaltsstoffe in einem geeigneten wässrigen, alkoholischen oder wässrig-alkoholischen Träger enthalten. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, eine pulverförmige oder auch Tabletten-förmige Formulierung bereitzustellen, was für Färbe- und/oder Aufhellmittel bevorzugt ist.

Unter wässrig-alkoholischen Lösungen sind beispielsweise wässrige Lösungen enthaltend 3 bis 70 Gew.-% eines C₁-C₄-Alkohols, insbesondere Ethanol bzw. Isopropanol, zu verstehen. Derartige wässrig-alkoholische Lösungen können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte Mittel sind dadurch gekennzeichnet, dass sie zusätzlich ein nichtwässriges Lösungsmittel enthalten, wobei besonders bevorzugte Mittel das Lösungsmittel in einer Konzentration von 0,1-30 Gewichtsprozent, bevorzugt in einer Konzentration von 1-20 Gewichtsprozent, ganz besonders bevorzugt in einer Konzentration von 2-10 Gewichtsprozent, jeweils bezogen auf das Mittel, enthalten.

In weiter bevorzugten Mitteln ist das Lösungsmittel ausgewählt aus Ethanol, n-Propanol, Isoropanol, n-Butanol, Propylenglykol, n-Butylenglykol, Glycerin, Diethylenglykolmonoethylether, Diethylenglykolmono-n-butylether, Phenoxyethanol und Benzylalkohol sowie ihren Mischungen.

Der pH-Wert der erfindungsgemäßen Mittel kann durch geeignete Inhaltsstoffe wie Acidifizierungsmittel oder Alkalisierungsmittel in einem weiten Bereich eingestellt werden.

Eine oxidative Färbung der keratinhaltigen Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt. Als Oxidationsmittel kommen Persulfate, Chlorite und insbesondere Wasserstoffperoxid oder dessen Anlagerungsprodukte in Frage, wie zuvor beschrieben.

Zusätzlich können die Mittel Metallionen oder Metallionenkomplexe enthalten, beispielsweise Cu-, Fe-, Mn- oder Ru-Ionen oder Komplexe dieser Ionen. Vorteilhaft ist weiterhin die Gegenwart von Komplexbildnern bei Zusatz dieser Metallionen. Die Komplexbildner können dabei ausgewählt werden aus Polycarbonsäuren, geminalen Diphosphonsäuren, Aminophosphonsäuren, Phosphonopolycarbonsäuren, Cyclodextrinen, Aminodicarbonsäuren, Polyacetalen oder Phosphonaten.

Bevorzugt werden die Mittel wasserarm oder wasserfrei formuliert. Bevorzugte Mittel sind dadurch gekennzeichnet, dass sie weniger als 5 Gew.-%, vorzugsweise weniger als 2 Gew.-%, besonders bevorzugt weniger als 1 Gew.-% und insbesondere weniger als 0,5 Gew.-% Wasser enthalten. Der Wassergehalt der Mittel lässt sich beispielsweise mittels Titration nach Karl Fischer bestimmen.

Auch ohne weitere Ausführungen wird davon ausgegangen, dass ein Fachmann die obige Beschreibung in weitestem Umfang nutzen kann. Die bevorzugten Ausführungsformen und Beispiele sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

Weitere wichtige Merkmale und Vorteile der Erfindung ergeben sich aus den Unteransprüchen und aus den Beispielen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsformen der Erfindung sind in den Beispielen dargestellt und werden in der nachfolgenden Beschreibung näher erläutert, ohne den Umfang der vorliegenden Erfindung zu beschränken.

### Beispiel 1: 1,2-O-(R,S)-(benzyliden)-α-D-glucofuranurono-6,3-lacton (Ic)

In einem Mehrhalskolben werden 22,7 g (166 mmol) Zinkchlorid und 171 mL (1,7 mol) Benzaldehyd vorgelegt. Nun werden 20,0 g (114 mmol) Glucuronolacton (MP Biomedicals, LLC) zugegeben und 3 Tage gerührt. Das Reaktionsgemisch wird mit Methyl-tert.-butyl-Ether (MtB-Ether) versetzt und mit Wasser extrahiert. Die separierte organische Phase wird über Natriumsulfat getrocknet, filtriert und im Vakuum das Lösungsmittel entfernt. Der Rückstand wird chromatographisch aufgereinigt und anschließend aus Ethylacetat/Heptan und Ethanol umkristallisiert. Man erhält eine Mischung von (R)/(S) im Verhältnis 68/10.

MS (EI): m/z (Relative Intensität, %) = 264,2 ([M+] 40)
¹H-NMR (δ₆-DMSO, 400MHz): δ = 4.7 (m, 1H), 5.0 (d, 1H, J=3.7Hz), 5.03 (d, 1H, *J*=3.05Hz), 6.12 (s, 1H), 6.16 (d, 1H, J=3,7Hz), 6.23 (d, 1OH, *J*=7Hz), 7.43-4.47 (m, 5H).

### Beispiel 2: 1-O-Benzyl-α-D-glucofuranurono-6,3-lacton (Id)

1,5 g (8,5 mmol) Glucuronolacton wird in 35,0 mL (340 mmol) Benzylalkohol und 630 µL (0,3 mmol) Salzsäure (w = 37%) gelöst und bei 80°C für 16 h gerührt. Das abgekühlte Reaktionsgemisch wird mit basischem Ionenaustauscher (Dowex 1-X8) neutralisiert und mit Heptan/Ethylacetat 2:1 chromatographiert.

MS (EI): m/z (Relative Intensität, %) = 266,25 ([M+] 2)
¹H-NMR (δ₆-DMSO, 400MHz): δ = 4.0 (dd, 1 H), 4.15 (s, 1 H), 4.32 (d, 1OH), 4.49 (d, 1H), 4.77 (m, 2H), 4.87 (dd, 1H), 5.05 (s, 1H), 7.24-7.30 (m, 5H), 12.8 (bs, 1OH).

### Beispiel 3: 1,2-O-(R,S)-(4-methoxybenzyliden)-α-D-glucofuranurono-6,3-lacton (le)

1,2 g (6,8 mmol) Glucuronolacton, 12,3 mL (102 mmol) 4-Methoxybenzaldehyd und 1,4 g (10,2 mmol) Zinkchlorid werden zusammen gegeben und bei RT 4 Wochen gerührt. Dann wird das Reaktionsgemisch mit MtB-Ether verdünnt und der Feststoff der Suspension abgesaugt. Die Mutterlauge wird mit Wasser versetzt und die Phasen getrennt. Die wässrige Phase wird mit MtB-Ether extrahiert, die vereinten organischen Phasen werden mit Wasser gewaschen, mit Natriumsulfat getrocknet, filtriert und im Vakuum das Lösungsmittel entfernt. Der Rückstand wird mit Heptan/Ethylacetat 1:2 chromatographiert. Man erhält eine Mischung von (R)/(S) im Verhältnis 27/10.

MS (EI): m/z (Relative Intensität, %) = 294,3 ([M+] 27)
¹H-NMR (CDCl₃, 500MHz): δ = 3.86 (s, 3H), 4.58 (d, 1H, J=4,5Hz), 4.99-5.04 (m, 1H, 1H, 1H), 6.05 (s, 1H), 6.19 (d, 1H, J=3,6Hz), 6.97, 7.42 (2d, 2x2H, J=8.5H), 7,31 (s, 1OH).

### Beispiel 4: 5-hydroxy-1,2-O-isopropyliden-α-D-glucofuranurono-6,3-lacton (If)

5,0 g (23,1 mmol) Glucuronolactonacetonid (Verbindung (la), kommerziell erhältlich) und 20,1 g (231 mmol) Mangan(IV)-oxid werden in 80,0 mL Aceton suspendiert und 24h bei RT gerührt. Das Reaktionsgemisch wird über einen Druckfilter (0,45 µm) filtriert, mit Aceton nachgewaschen und im Vakuum das überschüssige Aceton abdestilliert. Der erhaltene Feststoff wird mittels Kristallisation aus Wasser aufgereinigt.

MS (EI): m/z (Relative Intensität, %) = 213,8 ([M-H₂O] 1)
¹H-NMR (δ₆-DMSO, 400MHz): δ = 4.39 (d, 1 H, J=2.9Hz), 4,82 (d, 1 H, J=3.7Hz), 4,85 (d, 1H, J=2.9Hz), 4,95 (d, 1H, J=3.7Hz), 7,29 (s, 1OH), 7,46 (s, OH).

### Beispiel 5: 1,2-O-(R,S)-(4-hydroxy-3-methoxybenzyliden)-α-D-glucofuranurono-6,3-lacton (Ig)

15,4 g (102 mmol) Vanillin wird vorgelegt und bei 82°C geschmolzen. Bei dieser Temperatur werden nun 1,2 g (6,8 mmol) Glucuronolacton und 1,4 g (10,0 mmol) Zinkchlorid zugegeben und 16 h gerührt. Der nach Abkühlung entstehendene schwarze Sirup wird mit Ethylacetat und THF gelöst und dazu wird Wasser gegeben. Nun wird THF wieder im Vakuum abdestilliert und anschließend die Phasen getrennt. Die wässrige Phase wird mit Ethylacetat extrahiert, die vereinten organischen Phasen werden mit Wasser gewaschen, über Natriumsulfat getrocknet, filtriert und das Lösungsmittel im Vakuum abdestilliert. Aufreinigung erfolgt durch Chromatographie mit Heptan/Ethylacetat 1:2.

MS (EI): m/z (Relative Intensität, %) = 310,3 ([M+] 11)
¹H-NMR (CDCl₃, 500MHz): δ = 3.79 (s, 3H), 3.96 (dd, 1H), 4.19 (dd, 1H), 4.56 (dd, 1H), 4.65 (d, 1 H), 4.94 (d, 1 H), 5.49 (s, 1 H), 6.78 (dd, 1 H), 7.01 (dd, 1 H), 7.15 (dd, 1 H).

### Beispiel 6: 1-O-tetradecyl-α-D-glucofuranurono-6,3-lacton (Ih)

Zu einer Suspension aus 1,0 g (5,7 mmol) Glucuronolacton in 9,2 mL Tetrahydrofuran wird 2,4 g (11,4 mmol) 1-Tetradecanol und 2,1 mL (17,0 mmol) Bortrifluorid-Diethylether-Komplex zugegeben. Das Reaktionsgemisch wird 3 h bei Rückfluss gerührt und dann im Vakuum eingeengt. Der Rückstand wird chromatographisch mit Heptan/Ethylacetat aufgereinigt.

MS (EI): m/z (Relative Intensität, %) = 372,5 ([M+] 3)
¹H-NMR (δ₆-DMSO, 500MHz): δ = 0.86 (t, 3H), 1.26 (m, 20H), 1.40 (t, 2H), 3.22 (m, 1 H), 3.70 (m, 1 H), 4.07 (d, 1 H), 4.45 (t, 1 H), 4.73 (d, 1 H), 4.78 (t, 1 H), 4.93 (s, 1 H), 5.68 (d, 1H), 5.79 (d, 1 H).

### Beispiel 7: 1,2-O-(R,S)-(α-methytbenzyliden)-α-D-glucofuranurono-6,3-lacton (Ii)

Analog zu der Synthese der Verbindung (Ic) werden in einem Kolben 20,5 g (150 mmol) Zinkchlorid und 19,2 g (160 mmol) Acetophenon in 150 ml THF vorgelegt. Nun werden 17,5 g (100 mmol) Glucuronolacton zugegeben und 4 Tage bei 60°C gerührt. Man erhält die Verbindung (Ij).

### Beispiel 8: 1,2-O-(R,S)-(α-dibenzyliden)-α-D-glucofuranurono-6,3-lacton (Ij)

Analog zu der Synthese der Verbindung (Ic) werden in einem Kolben 20,5g (150 mmol) Zinkchlorid und 29,1 g (160 mmol) Benzophenon in 150 ml THF vorgelegt. Nun werden 17,5 g (100 mmol) Glucuronolacton zugegeben und 4 Tage bei 60°C gerührt. Man erhält die Verbindung (Ij).

### Bespiel 9: 1,2-O-(R,S)-(4-hydroxy-3-methoxycinnamyliden)-α-D-glucofuranurono-6,3-lactone (Ik)

Analog zu der Synthese der Verbindung (Id) werden in einem Kolben 5,7 g (41 mmol) Zinkchlorid und 6.23 g (35 mmol) 4-Hydroxy-3-methoxy-cinnamaldehyd in 150 mL THF vorgelegt. Nun werden 5,0 g (28.5 mmol) Glucuronolacton zugegeben und 2 Tage gerührt. Man erhält die Verbindung (Ik).

### Beispiel 10: 1-0-Cinnamyl-α-D-glucofuranurono-6,3-lacton (Im)

Analog zu der Synthese der Verbindung (Id) werden 1,0 g (5,7 mmol) Glucuronolacton in 9,2 mL Tetrahydrofuran mit 1,52 g (11,4 mmol) Zimtalkohol und 630 µL (0,3 mmol) Salzsäure (w = 37%) gelöst und bei 80°C für 16 h gerührt. Man erhält die Verbindung (Im).

### Anwendungsbeispiele:

### Beispiel A: Liquid Skin Model

In einen 100 mL Messkolben werden 1 mmol des jeweiligen Glucuronolacton-Derivates und 1 mmol (146 mg) L-Lysin eingewogen. Anschließend wird mit einem Gemisch aus 6 mL phosphat-gepufferten Wasser (pH = 7) und 94 mL Ethylenglycol aufgefüllt. Unter kräftigem Rühren wird die Reaktion beobachtet und die L*a*b*-Werte nach 24 Stunden bestimmt.

### Ergebnisse nach 24 Stunden:

| Verbindungen | L* | a* | b* |
|---|---|---|---|
| Verbindung Ia | 82,6 | 29,4 | 103,7 |
| Verbindung Ib | 81,1 | 30,8 | 103,8 |
| Verbindung Ic | 68,0 | 55,2 | 109,6 |
| Verbindung Ie | 76,4 | 39,1 | 106,7 |
| Verbindung Ig | 80,9 | 32,0 | 100,0 |
| Glucuronolactone | 96,1 | 4,3 | 45,9 |
| DHA | 46,7 | 27,2 | 69,4 |
| Erythrulose | 92,6 | 4,8 | 35,8 |

Nach 24 Stunden zeigen die Verbindungen (la), (Ib), (Ic), (Ie) und (Ig) niedrigere L-Werte als Erythrulose. Es bedeutet, dass diese Verbindungen eine dunklere Farbe im gewählten Testsystem im Vergleich zu Erythrulose erzeugen. Die a-Werte der erfindungsgemäßen Verbindungen sind höher als die a-Werte von DHA oder Erythrulose. Die Verbindungen nach Formel I sind daher in der Lage, einen Farbton zu erzielen, der mehr nach Rot verschoben ist.

### Beispiel B: Färbetest

Es werden je 1 mmol von DHA und den Glucuronolacton-Derivaten in 100 mL Erlenmeyer mit Schliff eingewogen:
(1) DHA: 90 mg
(2) Glucuronolacton: 176 mg
(3) Verbindung (la): 216 mg
(4) Verbindung (Ic): 264 mg

In jeden Erlenmeyerkolben werden nun ein Merinowollbällchen von der Größe einer Traube und ein Magnetrührer gegeben.

Dann wird eine Mischung aus 6 Vol% Puffer (ph7) und 94 Vol% Ethylenglycol hergestellt, in jeden Erlenmeyer werden 100 mL dieser Mischung gegeben und 24 h bei RT gerührt.

Nach 24 h werden die Wollbällchen erst mit Wasser, dann mit warmer Waschpulverlösung und wieder mit Wasser gewaschen und getrocknet.

Die Wollproben, die mit DHA oder Glucuronolacton behandelt wurden, zeigen keine Färbung gegenüber der unbehandelten Probe.

Die Wollproben, die mit der Verbindung (Ia) oder der Verbindung (Ic) behandelt wurden, zeigen eine gelbe Färbung.

Dieses Färbeergebnis lässt sich auch auf Haar übertragen, da Merinowolle, ebenso wie Haare, aus Proteinen (Keratinen) aufgebaut ist und die äußerste Schicht ist jeweils die Cuticula.

### Beispiel C: Stabilität/Geruch

Um den Geruch und die Stabilität von Glucuronolacton, Glucuronolactonacetonid der Formel (la), DHA und einer Kombination in einer Formulierung zu vergleichen, werden 5 Formulierungen hergestellt: Eine ohne Wirkstoff als Placebo, eine mit 5% Glucuronolacton, eine mit 5% Verbindung (la), eine mit 5% DHA und eine mit 5% DHA + 1 % Verbindung (la) (in der Rezeptur mit "Wirkstoff" bezeichnet).

Es wird folgende Rezeptur verwendet:

| Test Formulierung: | | | |
|---|---|---|---|
| **Bestandteile / Handelsname** | **Bezugsquelle** | **INCI** | **[Gew-%]** |
| **A** | | | |
| Miglyol 812 N | (1) | CAPRYLIC/CAPRIC TRIGLYCERIDE | 15 |
| Finsolv TN | (2) | CETIOL AB | 2.5 |
| Pionier COLD | (3) | O/W- | 2.5 |
| | | HAUTCREMEGRUNDLAGE | |
| **B** | | | |
| 1,2-Propandiol | (4) | PROPYLEN GLYCOL | 4 |
| Wasser, demineralisiert | | AQUA (WATER) | 40 |
| | | | |
| **C** | | | |
| Pionier NP-37G | (3) | VERDICKUNGSMITTEL | 0.5 |
| Wasser, demineralisiert | | | 20 |
| | | | |
| D | | | |
| Wirkstoff | (4)/(5) | | Siehe |
| | | | oben |
| Wasser, demineralisiert | | AQUA (WATER) | Ad 100 |

### Bezugsquellen:

(1) Sasol Germany GmbH, (2) Cognis GmbH, (3) Hansen&Rosenthal KG, (4) Merck KGaA/Rona®, (5) Alfa Aesar

### Herstellung der Formulierungen:

Phase A erhitzen, Phase B erhitzen, Phase C auf dem Magnetrührer herstellen (erst Wasser vorlegen, dann Verdicker dazu), dann Phase C mit Phase B mit dem Ultra-Turrax homogenisieren und dabei langsam Phase A dazugeben. Anschließend wird Phase D dazugegeben und ebenfalls gut homogenisiert.

Bei der 5. Creme (mit DHA und Glucuronolactonacetonid) wird der Wasseranteil entsprechend angeglichen.

### Ergebnis:

In den Formulierungen, die bei 40°C 8 Wochen lang gelagert wurden, änderte sich mit der Zeit die Farbe (außer beim Placebo). Die Formulierungen enthaltend DHA und die Mischung DHA mit Verbindung la entwickelten den typischen DHA-Geruch, allerdings bei der Mischung DHA mit Verbindung la deutlich schwächer als bei DHA allein. Dieser schwächere Geruch korreliert mit einer helleren Farbe der Formulierung (in Vergleich mit der Farbe der Formulierung enthaltend DHA allein). Die Formulierungen enthaltend Glucuronolacton allein und Verbindung la haben eine ähnliche Farbe als die Formulierung enthaltend die Mischung DHA mit Verbindung la, erzeugen aber keinen Geruch.

### Beispiel D: ex-vivo-Studie

Aus dem Unterleibsgewebe einer 50 jährigen europäischen Frau werden 24 histologische Explantate mit einem mittleren Durchmesser von 10 mm vorbereitet. Die Gewebestücke werden in BEM Medium(BIO-EC's Explants Medium) bei 37°C in einer feuchten Atmosphäre gehalten, die 5 %-CO₂ enthält.

Eingeteilt wird das Gewebe in 8 Chargen von je 3 Explantaten:

| | |
|---|---|
| Eine Negativkontrolle (unbehandelte Probe, nur mit dem Träger Mygliol/Ethanol (8/2) behandelt) | → T |
| Eine Positivkontrolle (Dihydroxyaceton 1%) | → R1 |
| Eine Positivkontrolle (Erythrulose 4%) | → R2 |
| Glucuronolacton (1%) | → P1 |
| Verbindung der Formel (la) (1%) | → P2-1 |
| Verbindung der Formel (la) (4%) | → P2-2 |

Es werden 30 µL der Substanz-Lösungen an 5 aufeinander folgenden Tagen unter Verwendung eines runden Filterpapiers appliziert und 2 h einwirken lassen. An den Tagen 0, 5 und 8 werden aus den Explantaten 5µm dicke Gewebeschnitte hergestellt. Diese Schnitte werden morphologisch untersucht und chromametrisch vermessen.

Die Farbmessungen werden mit einem Chroma-Meter der Firma Minolta CR-300 durchgeführt und die L-, a- und b-Werte entsprechend am Gerät abgelesen.

### Prinzip der chromametrischen Messung:

Es werden die folgenden Parameter eingestellt:
Im Jahre 1931 wurde durch die CIE (Commision Internationale de L'Eclairage) eine Testreihe durchgeführt, wobei der "2-Grad-Normalbeobachter" definiert wurde. Dabei liegt eine Farbfläche vor, die mit einem Blickwinkel von 2 Grad gesehen wird. Dieser Blickwinkel wird als Standard bei der chromametrischen Messung benutzt.
   - Bestrahlungsquelle D65, entsprechend Tageslicht
   - Gebiet der Messung: 3 mm
   - Benutzte Parameter: L* a* b*

Die Probe wird mit der Epidermis nach unten auf die Auflageschiene aufgelegt. Die Bestrahlung erfolgt von der Unterseite durch das angegebene Areal (3 mm).

### Kontraststudie:

Bei den kolorimetrischen Bestimmungen « L a b » bestimmt der L-Wert den Kontrast der Haut. Sinkt der L-Wert, erscheint die Haut dunkler.

ITA ist definiert als arctang[(L-50)/b]. Erhöht sich der ITA Index, erscheint die Haut heller.

### Ergebnisse:

| | Tag 5 (ITA-Wert) |
|---|---|
| (T) | +2% |
| (R1) | -15% |
| (R2) | -5% |
| (P1) | -3% |
| (P2-1) | -2% |
| (P2-2) | -5% |

### Formulierungsbeispiel 1: O/W Bräunungscreme

| **Bestandteile / Handelsname** | **Bezugsquelle** | **INCI** | **[Gew%]** |
|---|---|---|---|
| **A** | | | |
| Tego Care 150 | (1) | GLYCERYL STEARATE, STEARETH-25-CETETH-20, STEARYL ALCOHOL | 8 |
| Miglyol 812 N | (2) | CAPRYLIC/CAPRIC TRIGLYCERIDE | 3 |
| Isopropyl myristat | (3) | ISOPROPYL MYRISTATE | 2 |
| Paraffin liquid | (4) | PARAFFINUM LIQUIDUM (MINERAL OIL) | 12 |
| Paraffin | (4) | PARAFFIN | 2 |
| Propyl-4-hydroxybenzoat | (4) | PROPYLPARABEN | 0.15 |
| **Verbindung (Ic)** | | | 5 |
| | | | |
| **B** | | | |
| 1,2-Propandiol | (4) | PROPYLENE GLYCOL | 4 |
| Sorbitol F liquid | (4) | SORBITOL | 2 |
| Methyl-4-hydroxybenzoat | (4) | METHYLPARABEN | 0.05 |
| Water, demineralisiert | | AQUA (WATER) | ad 100 |
| | | | |
| **C** | | | |
| Wasser, demineralisiert | | | 11.8 |
| **D** | | | |
| Parfum (q.s.) | | PARFUM | 0.50 |
| **Gesamt** | | | **100.00** |

### Herstellungsverfahren:

Zunächst werden die Phasen A und B getrennt auf 75°C erwärmt. Danach wird Phase A unter vorsichtigem Rühren langsam zu Phase B gegeben. Es wird bei 65 °C für eine Minute homogenisiert. Anschließend wird unter Rühren auf 40 °C abgekühlt und die Phase C unter Rühren zugegeben, abgekühlt auf 35°C und die Phase D zugegeben, und weiter abgekühlt.

### Bezugsquellen:

(1) Degussa-Goldschmidt AG (2) Sasol Germany GmbH (3) Cognis GmbH (4) Merck KGaA/Rona®

### Formulierungsbeispiel 2: ONV Bräunungscreme

| **Bestandteile / Handelsname** | **Bezugsquelle** | **INCI** | **[Gew-%]** |
|---|---|---|---|
| **A** | | | |
| Tego Care 150 | (1) | GLYCERYL STEARATE, STEARETH-25, CETETH-20, STEARYL ALCOHOL | 8 |
| Lanette O | (2) | CETEARYL ALCOHOL | 1.5 |
| Luvitol EHO | (3) | CETEARYL OCTANOATE | 5 |
| Miglyol 812 N | (4) | CAPRYLIC/CAPRIC TRIGLYCERIDE | 5 |
| Paraffin liquid | (5) | PARAFFINUM LIQUIDUM (MINERAL OIL) | 3 |
| AbilWax 2434 | (1) | STEAROXY DIMETHICONE | 1.6 |
| Dow Corning 200 Fluid (350 cs) | (6) | DIMETHICONE | 0.5 |
| Propyl-4-hydroxybenzoate | (5) | PROPYLLPARABEN | 0.05 |
| **B** | | | |
| 1,2-Propanediol | (5) | PROPYLENE GLYCOL | 3 |
| Methyl-4-hydroxybenzoate | (5) | METHYLPARABEN | 0.15 |
| Water, demineralized | | AQUA (WATER) | ad 100 |
| **C** | | | |
| Dihydroxyacetone | (8) | DIHYDROXYACETONE | 5 |
| Probiol L 05018 (Empty liposomes) | (7) | AQUA, ALCOHOL DENAT, LECITHIN, GLYCERINE, DISODIUM PHOSPHATE | 5 |
| Water, demineralized | | AQUA (WATER) | 10.00 |
| **Verbindung (Ia)** | (9) | | 5 |
| | | | |
| **Gesamt** | | | **100.00** |

### Herstellungsverfahren:

Zunächst werden die Phasen A und B auf 80 °C erwärmt. Danach wird Phase B unter Rühren langsam zu Phase A gegeben und homogenisiert. Dann wird abgekühlt und die Phase C bei 40°C zugegeben.

### Bezugsquellen:

(1) Degussa-Goldschmidt AG, (2) Cognis GmbH, (3) BASF AG, (4) Sasol Germany GmbH, (5) Merck KGaA/Rona®, (6) Dow Corning(7) Kuhs GmbH & Co. KG, (8) Merck KGaA/Rona®, (9) Alfa Aesar

### Formulierungsbeispiel 3: O/W Bräunungslotion

| **Bestandteile / Handelsname** | **Bezugsquelle** | **INCI** | **[Gew%]** |
|---|---|---|---|
| **A** | | | |
| Montanov 68 | (1) | CETEARYL ALCOHOL, CETEARYL GLUCOSIDE | 4 |
| Span 60 | (2) | SORBITAN STEARATE | 1.5 |
| Lanette O | (3) | CETEARYL ALCOHOL | 1 |
| Cosmacol ELI | (4) | C12-13 ALIKYL LACTATE | 2 |
| Arimol HD | (2) | ISOHEXADECANE | 1 |
| Paraffin highly liquid | (5) | PARAFFINUM LIQUIDUM (MINERAL OIL) | 3 |
| Dow Corning 9050 Silicone Elastomer Blend | (6) | CYCLOMETHICONE, DIMETHICONE CROSSPOLYMER | 2 |
| RonaCare^{®} Tocopherol Acetate | (5) | TOCOPHERYL ACETATE | 0.5 |
| Propyl-4-hydroxybenzoate | (5) | PROPYLPARABEN | 0.05 |
| | | | |
| **B** | | | |
| RonaCare^{®}Ectoin | (5) | ECTOIN | 0.5 |
| Glycerol, anhydrous | (5) | GLYCERINE | 2 |
| Water, demineralized | | AQUA (WATER) | ad 100 |
| Methyl-4-hydroxybenzoate | (5) | METHYLPARABEN | 0.15 |
| | | | |
| **C** | | | |
| Rhodicare S | (7) | XANTHAN GUM | 0.2 |
| | | | |
| **D** | | | |
| | | | |
| Probiol L 05018 (Empty liposomes) | (8) | AQUA, ALCOHOL DENAT, LECITHIN, GLYCERINE, DISODIUM PHOSPHATE | 5 |
| **Verbindung (Ic)** | | | 5 |
| Water, demineralized | | AQUA (WATER) | 10 |
| E | | | |
| Fragrance Cucumber | (9) | PARFUM | 0.2 |
| | | | |
| **Gesamt** | | | **100.00** |

### Herstellungsverfahren:

Zunächst werden die Phasen A und B separat gemischt und auf 75 °C erwärmt. Danach wird Phase C in Phase B gegeben und unter Rühren zu Phase A gegeben. Es wird homogenisiert. Dann wird unter Rühren abgekühlt und die Phasen D und E bei 40°C zugegeben.

### Bezugsquellen:

(1) Seppic (2) Uniqema (3) Cognis GmbH
(4) Condea Chinica D.A.C.S.p.A. (5) Merck KGaA/Rona® (6) Dow Corning (7) Rhodia GmbH (8) Kuhs GmbH & Co. KG (9) Drom

### Formulierungsbeispiel 4: milde transparente W/O Bräunungslotion

| **Bestandteile / Handelsname** | **Bezugsquelle** | **INCI** | **[Gew-%]** |
|---|---|---|---|
| **A** | | | |
| Dow Corning 3225 C | (1) | CYCLOMETHICONE, DIMETHICONE COPOLYOL | 23.6 |
| Propyl-4-hydroxybenzoate | (2) | PROPYLPARABEN | 0.05 |
| | | | |
| **B** | | | |
| Dihydroxyacetone | (3) | DIHYDROXYACETONE | 3 |
| **Verbindung (Ia)** | (4) | | 2 |
| Methyl-4-hydroxybenzoate | (2) | METHYLPARABEN | 0.15 |
| 1,2-Propanediol | (2) | PROPYLENE GLYCOL | 35.9 |
| Water, demineralized | | AQUA (WATER) | ad 100 |
| | | | |
| **Gesamt** | | | **100.00** |

### Herstellungsverfahren:

Zunächst wird die Phase B aufgelöst und dann wird sie zu Phase A gegeben. Der pH-Wert wird mit Natronlauge bzw. Citronensäure auf den Wert pH = 6.0 eingestellt.

### Bezugsquellen:

(1) Dow Corning (2) Merck KGaA/Rona® (3) Merck KGaA/Rona® (4) Alfa Aesar

### Formulierungsbeispiel 5: O/W Bräunungscreme mit UV A/B Schutz

| **Bestandteile / Handelsname** | **Bezugsquelle** | **INCI** | **[Gew-%]** |
|---|---|---|---|
| **A** | | | |
| | | | |
| Eusolex^{®} 2292 | (1) | ETHYLHEXYL METHOXYCINNAMATE, BHT | 3 |
| Eusolex^{®} 4360 | (1) | BENZOPHENONE-3 | 0.5 |
| Tego Care 150 | (2) | GLYCERYL STEARATE, STEARETH-25, CETETH-20, STEARYL ALCOHOL | 8 |
| Lanette O | (3) | CETEARYL ALCOHOL | 1.5 |
| Luvitol EHO | (4) | CETEARYL OCTANOATE | 5 |
| Miglyoll 812 N | (5) | CAPRYLIC/CAPRIC TRIGLYCERIDE | 5 |
| Paraffin liquid | (1) | PARAFFINUM LIQUIDUM (MINERAL OIL) | 3 |
| Abil-Wax 2434 | (2) | STEAROXY DIMETHICONE | 1.6 |
| Dow Corning 200 Fluid (350 cs) | (6) | DIMETHICONE | 0.5 |
| Propyl-4-hydroxybenzoate | (1) | PROPYLPARABEN | 0.05 |
| **B** | | | |
| 1,2-Propanediol | (1) | PROPYLENE GLYCOL | 3 |
| Methyl-4-hydroxybenzoate sodium salt | (1) | SODIUM METHYLPARABEN | 0.17 |
| Wasser, demineralisiert | | AQUA (WATER) | ad 100 |
| **Verbindung (Ic)** | | | 5 |
| C | | | |
| Dihydroxyaceton | (7) | DIHYDROXYACETONE | 5 |
| Wasser, demineralisiert | | | 10 |
| **Gesamt** | | | **100.00** |

### Herstellungsverfahren:

Zunächst werden die Phasen A und B getrennt gemischt und auf 80°C erwärmt. Danach wird Phase B langsam unter Rühren zu Phase A gegeben. Es wird homogenisiert und auf 40°C gekühlt und Phase C zugegeben, dann auf Raumtemperatur abgekühlt.

### Bezugsquellen:

(1) Merck KGaA/Rona®(2) Degussa-Goldschmidt AG (3) Cognis GmbH
(4) BASF AG(5) Sasol Germany GmbH (6) Dow Corning
(7) Merck KGaA/Rona®

### Formulierungsbeispiel 6: O/W schimmernde Bräunungslotion

| **Bestandteile / Handelsname** | **Bezugsquelle** | **INCI** | **[Gew-%]** |
|---|---|---|---|
| **A** | | | |
| Montanov 68 | (1) | CETEARYL ALCOHOL, CETEARYL GLUCOSIDE | 4 |
| Span 60 | (2) | SORBITAN STEARATE | 1.5 |
| Lanette O | (3) | CETEARYL ALCOHOL | 1 |
| Cosmacol ELI | (4) | C12-13 ALKYL LACTATE | 3 |
| Cosmacol EMI | (4) | DI-C12-13 ALKYL MALATE | 1 |
| Dow Corning 9040 Silicone Elastomer Blend | (5) | CYCLOMETHICONE, DIMETHICONE CROSSPOLYMER | 1 |
| Arlamol HD | (2) | ISOHEXADECANE | 3 |
| RonaCare^{®} Tocopherol Acetate | (6) | TOCOPHERYL ACETATE | 0.5 |
| Propyl-4-hydroxybenzoate | (6) | PROPYLPARABEN | 0.05 |

| B | | | |
|---|---|---|---|
| RonaCare^{®} Ectoin | (6) | ECTOIN | 0.5 |
| Colorona^{®} Red Gold | (6) | MICA, CI 77891 (TITANIUM DIOXIDE), CI 77491(ION OXIDES) | 2 |
| Glycerol, anhydrous | (6) | GLYCERIN | 2 |
| Caramel 250 | (7) | CARAMEL | 1 |
| FD&C Yellow No6 W082 | (8) | CI 15985 | 0.01 |
| Water, demineralized | | AQUA (WATER) | ad 100 |
| Methyl-4-hydroxybenzoate | (6) | METHYLPARABEN | 0.15 |
| **Verbindung Ia** | (11) | | 5 |

| **C** | | | |
|---|---|---|---|
| Sepigel 305 | (1) | LAURETH-7, POLYACRYLAMIDE, C13-14 ISOPARAFFIN | 0.5 |

| D | | | |
|---|---|---|---|
| Dihydroxyaceton | (10) | DIHYDROXYACETONE | 5 |
| Wasser, demineralisiert | | | 10 |

| E | | | |
|---|---|---|---|
| Fragrance Babylon | (9) | PARFUM | 0.2 |
| Gesamt | | | **100.00** |

### Herstellungsverfahren:

Zunächst werden die Phasen A und B getrennt auf 75 °C erwärmt. Danach wird Phase A langsam unter Rühren zu Phase B gegeben. Bei 60 °C wird Phase C zu A/B gegeben und es wird homogenisiert. Anschließend wird auf 40 °C abgekühlt und die Phasen D und E werden sukzessive zugegeben.

### Bezugsquellen:

(1) Seppic (2) Uniqema (3) Cognis GmbH
(4) Condea Chimica D.A.C.S.p.A. (5) Dow Corning (6) Merck KGaA/Rona®
(7) D. D. Williamson (8) Les Colorants Wackherr SA (9) Drom (10) Merck KGaA/Rona® (11) Alfa Aesar

## Patentansprüche

1. Verwendung mindestens einer Verbindung der Formel I 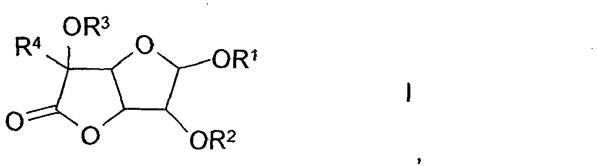
wobei R¹ und R² jeweils unabhängig voneinander Aryl,
eine geradkettige oder verzweigte Alkylgruppe mit 1 bis 30 C-Atomen, die mit Aryl substituiert sein kann oder
eine geradkettige oder verzweigte Alkenylgruppe mit 2 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
a) durch mindestens eine geradkettige oder verzweigte Alkylgruppe mit 1 bis 30 C-Atomen und/oder
b) durch mindestens eine Arylgruppe mit 6 bis 12 C-Atomen und/oder
c) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
Alkyl eine geradkettige oder verzweigte Alkylgruppe mit 1 bis 30 C-Atomen bedeutet,
und/oder deren Salze, Tautomere, Stereoisomere und/oder Solvate, einschließlich deren Mischungen in allen Verhältnissen als Selbstbräunungssubstanz.

2. Verwendung mindestens einer Verbindung der Formel I 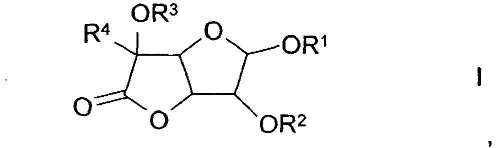
wobei R¹ und R² jeweils unabhängig voneinander Aryl,
eine geradkettige oder verzweigte Alkylgruppe mit 1 bis 30 C-Atomen, die mit Aryl substituiert sein kann oder
eine geradkettige oder verzweigte Alkenylgruppe mit 2 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
a) durch mindestens eine geradkettige oder verzweigte Alkylgruppe mit 1 bis 30 C-Atomen und/oder
b) durch mindestens eine Arylgruppe mit 6 bis 12 C-Atomen und/oder
c) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
Alkyl eine geradkettige oder verzweigte Alkylgruppe mit 1 bis 30 C-Atomen bedeutet,
und/oder deren Salze, Tautomere, Stereoisomere und/oder Solvate, einschließlich deren Mischungen in allen Verhältnissen als Bräunungsverstärker für Dihydroxyaceton oder für eine Mischung von Selbstbräunungssubstanzen enthaltend Dihydroxyaceton.

3. Verwendung mindestens einer Verbindung der Formel I 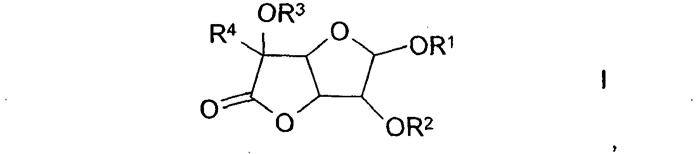
wobei R¹ und R² jeweils unabhängig voneinander Aryl,
eine geradkettige oder verzweigte Alkylgruppe mit 2 bis 30 C-Atomen, die mit Aryl substituiert sein kann oder
eine geradkettige oder verzweigte Alkenylgruppe mit 1 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
a) durch mindestens eine geradkettige oder verzweigte Alkylgruppe mit 1 bis 30 C-Atomen und/oder
b) durch mindestens eine Arylgruppe mit 6 bis 12 C-Atomen und/oder
c) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
Alkyl eine geradkettige oder verzweigte Alkylgruppe mit 1 bis 30 C-Atomen bedeutet,
und/oder deren Salze, Tautomere, Stereoisomere und/oder Solvate, einschließlich deren Mischungen in allen Verhältnissen zur Modulation des Farbtones, der bei der Bräunung mit Dihydroxyaceton oder durch die Mischung oder Zubereitung enthaltend Dihydroxyaceton erzielt wird.

4. Verwendung mindestens einer Verbindung der Formel I 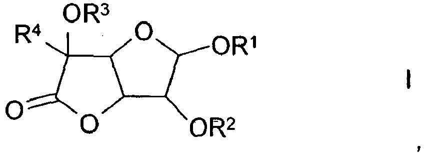
wobei R¹ und R² jeweils unabhängig voneinander Aryl,
eine geradkettige oder verzweigte Alkylgruppe mit 1 bis 30 C-Atomen, die mit Aryl substituiert sein kann oder
eine geradkettige oder verzweigte Alkenylgruppe mit 2 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
a) durch mindestens eine geradkettige oder verzweigte Alkylgruppe mit 1 bis 30 C-Atomen und/oder
b) durch mindestens eine Arylgruppe mit 6 bis 12 C-Atomen und/oder
c) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
Alkyl eine geradkettige oder verzweigte Alkylgruppe mit 1 bis 30 C-Atomen bedeutet,
und/oder deren Salze, Tautomere, Stereoisomere und/oder Solvate, einschließlich deren Mischungen in allen Verhältnissen als Kontrastreduktionsmittel.

5. Verwendung mindestens einer Verbindung der Formel I 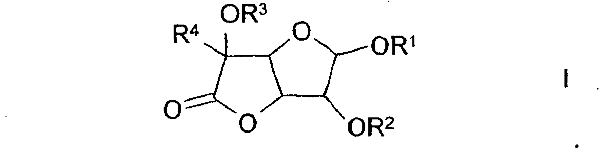
wobei R¹ und R² jeweils unabhängig voneinander Aryl,
eine geradkettige oder verzweigte Alkylgruppe mit 1 bis 30 C-Atomen, die mit Aryl substituiert sein kann oder
eine geradkettige oder verzweigte Alkenylgruppe mit 2 bis 30 C-Atomen, enthaltend eine oder mehrere Doppelbindungen, die mit Aryl substituiert sein kann, bedeuten,
wobei entweder R¹ oder R² H bedeuten kann,
wobei R¹ und R² zusammen auch einen unsubstituierten oder substituierten fünfgliedrigen Ring bilden können, der
g) durch mindestens eine geradkettige oder verzweigte Alkylgruppe mit 1 bis 30 C-Atomen und/oder
h) durch mindestens eine Arylgruppe mit 6 bis 12 C-Atomen und/oder
i) durch mindestens eine geradkettige oder verzweigte Alkenylgruppe mit 2 bis 30 C-Atomen enthaltend eine oder mehrere Doppelbindungen substituiert sein kann,
wobei die Alkylgruppe mit 1 bis 30 C-Atomen und/oder die Alkenylgruppe mit 1 bis 30 C-Atomen mit Aryl subsituiert sein kann und/oder die Arylgruppe mit 6 bis 12 C-Atomen durch Alkyl, OH, OAlkyl, NH₂, NH-Alkyl, N(Alkyl)₂, C(O)Alkyl, O-C(O)Alkyl und/oder C(O)-OAlkyl substituiert sein kann,
R³ H oder eine geradkettige oder verzweigte Alkylgruppe mit 1 bis 30 C-Atomen bedeutet und
R⁴ H oder OR³ bedeutet,
wobei
Aryl eine Arylgruppe mit 6 bis 12 C-Atomen bedeutet, die gegebenenfalls durch Alkyl, OH, OAlkyl, NH₂, NH-Alkyl, N(Alkyl)₂, C(O)Alkyl, O-C(O)Alkyl oder C(O)-OAlkyl substituiert sein kann, und
Alkyl eine geradkettige oder verzweigte Alkylgruppe mit 1 bis 30 C-Atomen bedeutet,
und/oder deren Salze, Tautomere, Stereoisomere und/oder Solvate, einschließlich deren Mischungen in allen Verhältnissen zur Färbung von keratinhaltigen Fasern.

6. Verwendung nach einem oder mehreren der Ansprüche 1 bis 5, **dadurch gekennzeichnet, dass** in der Verbindung der Formel I der Substituent R⁴ H bedeutet.

7. Verwendung nach einem oder mehreren der Ansprüche 1 bis 6, **dadurch gekennzeichnet, dass** in der Verbindung der Formel I der Substituent R³ H bedeutet.

8. Verwendung nach einem oder mehreren der Ansprüche 1 bis 7, **dadurch gekennzeichnet, dass** in der Verbindung der Formel I die Substituenten R¹ und R² zusammen einen fünfgliedrigen Ring bilden, der durch mindestens eine geradkettige oder verzweigte Alkylgruppe mit 1 bis 10 C-Atomen substituiert sein kann
und/oder durch mindestens eine geradkettige oder verzweigte Alkenylgruppe mit 1 bis 10 C-Atomen enthaltend eine oder mehrere Doppelbindungen substituiert sein kann, die durch mindestens eine Phenylgruppe substituiert sein kann, wobei die Phenylgruppe durch Alkyl, OH, OAlkyl, C(O)Alkyl, O-C(O)Alkyl und/oder C(O)-OAlkyl substituiert sein kann
und/oder durch eine Phenylgruppe substituiert sein kann, wobei die Phenylgruppe durch Alkyl, OH, OAlkyl, C(O)Alkyl, O-C(O)Alkyl und/oder C(O)-OAlkyl substituiert sein kann.

9. Verwendung nach einem oder mehreren der Ansprüche 1 bis 5, **dadurch gekennzeichnet, dass** die Verbindung der Formel I ausgewählt wird aus den Verbindungen (la) bis (Im)

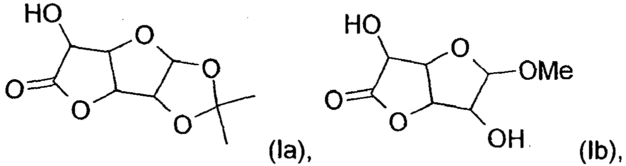

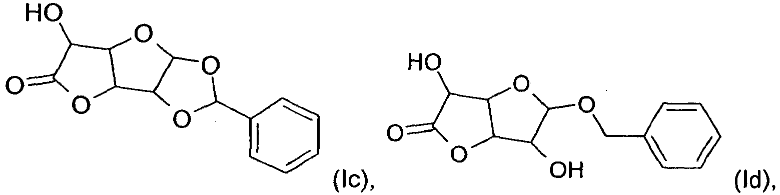

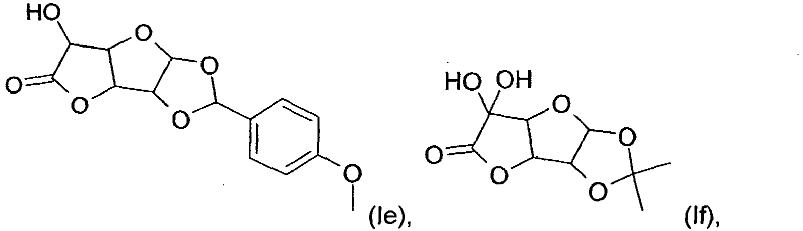

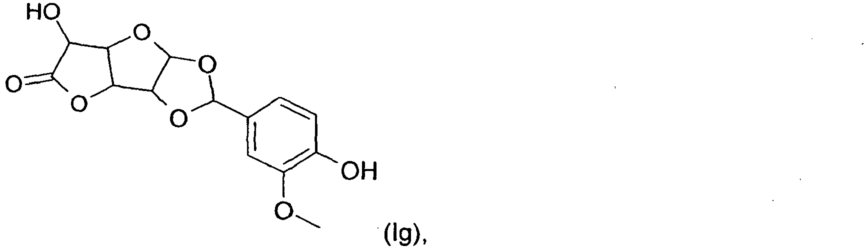

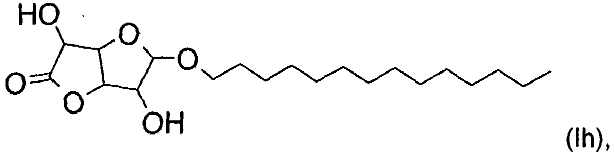

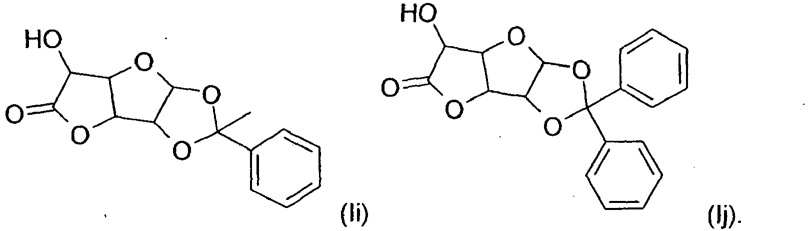

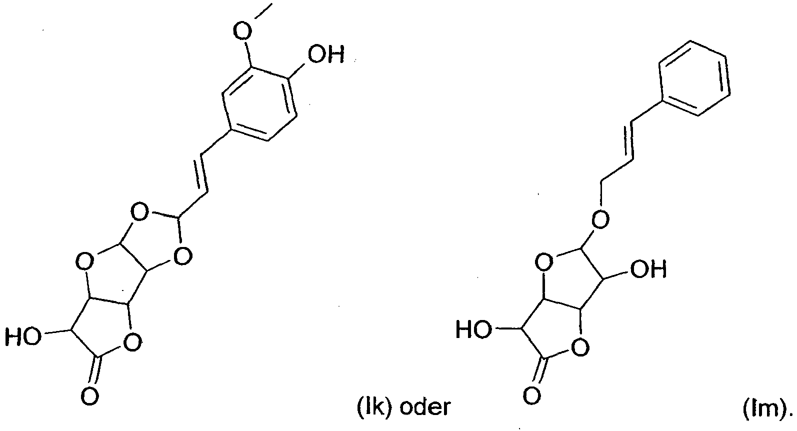

10. Zubereitung enthaltend Dihydroxyaceton und mindestens eine Verbindung nach Formel I gemäß einem oder mehreren der Ansprüche 1 bis 9 und/oder deren Salze, Tautomere, Stereoisomere und/oder Solvate, einschließlich deren Mischungen in allen Verhältnissen.

11. Zubereitung nach Anspruch 10, **dadurch gekennzeichnet, dass** die zumindest eine Verbindung der Formel I in einer Menge von 0,01 bis 20 Gew.-% enthalten ist, bezogen auf die Gesamtmenge der Zubereitung.

12. Zubereitung nach Anspruch 10 oder 11, **dadurch gekennzeichnet, dass** ein für kosmetische, pharmazeutische, dermatologische Zubereitungen geeigneter Träger enthalten ist.

13. Verfahren zur Herstellung einer Zubereitung nach Anspruch 12, **dadurch gekennzeichnet, dass** die zumindest eine Verbindung nach Formel I zusammen mit zumindest einem für kosmetische, pharmazeutische, dermatologische Zubereitungen geeigneten Träger und gegebenenfalls Hilfsstoffen und/oder Füllstoffen oder Selbstbräunungssubstanzen gemischt wird und Dihydroxyaceton zugegeben wird.

14. Mittel zum Färben von keratinhaltigen Fasern, **dadurch gekennzeichnet, dass** mindestens eine Verbindung der Formel I gemäß einem oder mehreren der Ansprüche 5 bis 9 und/oder deren Salze, Tautomere, Stereoisomere und/oder Solvate, einschließlich deren Mischungen in allen Verhältnissen, enthalten ist.

15. Mittel nach Anspruch 14, **dadurch gekennzeichnet, dass** ein weiterer direktziehender Farbstoff oder mindestens ein Oxidationsfarbstoffvorprodukt enthalten ist.

16. Verbindungen der Formeln

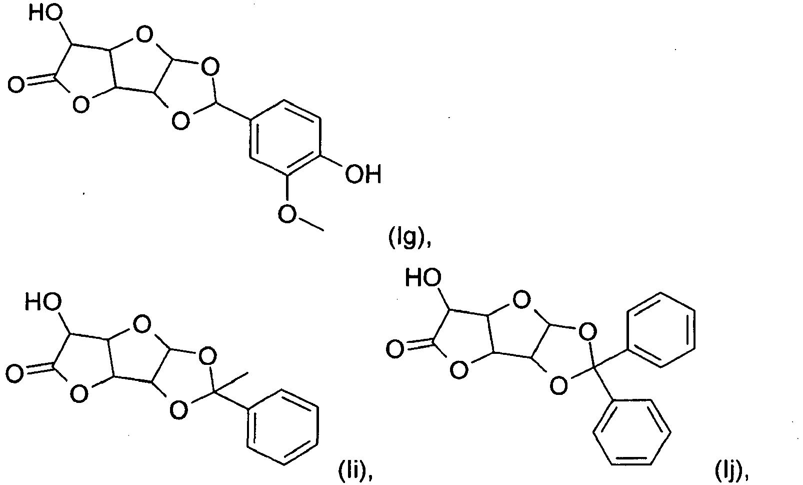

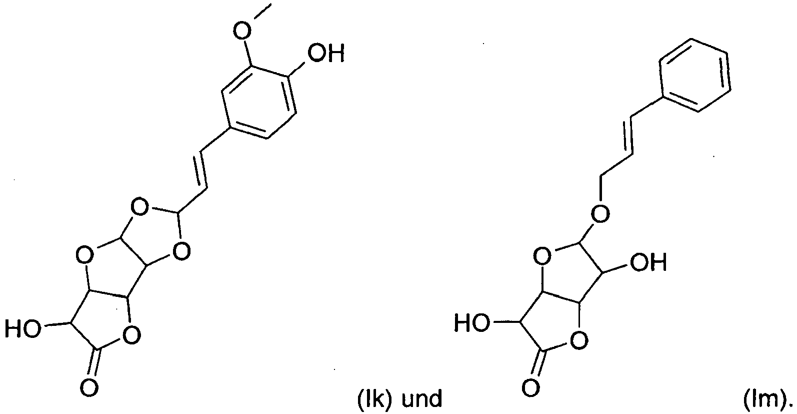

## Claims

1. Use of at least one compound of the formula I 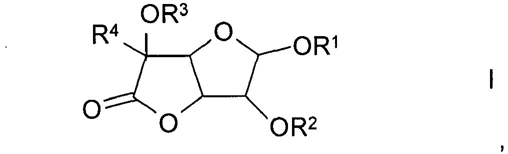
where R¹ and R² each, independently of one another, denote aryl,
a straight-chain or branched alkyl group having 1 to 30 C atoms, which may be substituted by aryl, or
a straight-chain or branched alkenyl group having 2 to 30 C atoms, containing one or more double bonds, which may be substituted by aryl,
where either R¹ or R² may denote H,
where R¹ and R² together may also form an unsubstituted or substituted five-membered ring, which may be substituted
a) by at least one straight-chain or branched alkyl group having 1 to 30 C atoms and/or
b) by at least one aryl group having 6 to 12 C atoms and/or
c) by at least one straight-chain or branched alkenyl group having 2 to 30 C atoms containing one or more double bonds,
where the alkyl group having 1 to 30 C atoms and/or the alkenyl group having 1 to 30 C atoms may be substituted by aryl and/or the aryl group having 6 to 12 C atoms may be substituted by alkyl, OH, Oalkyl, NH₂, NH-alkyl, N(alkyl)₂, C(O)alkyl, O-C(O)alkyl and/or C(O)-Oalkyl,
R³ denotes H or a straight-chain or branched alkyl group having 1 to 30 C atoms and
R⁴ denotes H or OR³,
where
aryl denotes an aryl group having 6 to 12 C atoms, which may optionally be substituted by alkyl, OH, Oalkyl, NH₂, NH-alkyl, N(alkyl)₂, C(O)alkyl, O-C(O)alkyl or C(O)-Oalkyl, and
alkyl denotes a straight-chain or branched alkyl group having 1 to 30 C atoms,
and/or salts, tautomers, stereoisomers and/or solvates thereof, including mixtures thereof in all ratios, as self-tanning substance.

2. Use of at least one compound of the formula I 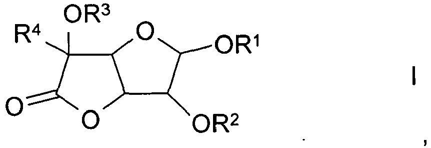
where R¹ and R² each, independently of one another, denote aryl,
a straight-chain or branched alkyl group having 1 to 30 C atoms, which may be substituted by aryl, or
a straight-chain or branched alkenyl group having 2 to 30 C atoms, containing one or more double bonds, which may be substituted by aryl,
where either R¹ or R² may denote H,
where R¹ and R² together may also form an unsubstituted or substituted five-membered ring, which may be substituted
a) by at least one straight-chain or branched alkyl group having 1 to 30 C atoms and/or
b) by at least one aryl group having 6 to 12 C atoms and/or
c) by at least one straight-chain or branched alkenyl group having 2 to 30 C atoms containing one or more double bonds,
where the alkyl group having 1 to 30 C atoms and/or the alkenyl group having 1 to 30 C atoms may be substituted by aryl and/or the aryl group having 6 to 12 C atoms may be substituted by alkyl, OH, Oalkyl, NH₂, NH-alkyl, N(alkyl)₂, C(O)alkyl, O-C(O)alkyl and/or C(O)-Oalkyl,
R³ denotes H or a straight-chain or branched alkyl group having 1 to 30 C atoms and
R⁴ denotes H or OR³,
where
aryl denotes an aryl group having 6 to 12 C atoms, which may optionally be substituted by alkyl, OH, Oalkyl, NH₂, NH-alkyl, N(alkyl)₂, C(O)alkyl, O-C(O)alkyl or C(O)-Oalkyl, and
alkyl denotes a straight-chain or branched alkyl group having 1 to 30 C atoms,
and/or salts, tautomers, stereoisomers and/or solvates thereof, including mixtures thereof in all ratios, as tanning enhancer for dihydroxyacetone or for a mixture of self-tanning substances comprising dihydroxyacetone.

3. Use of at least one compound of the formula I 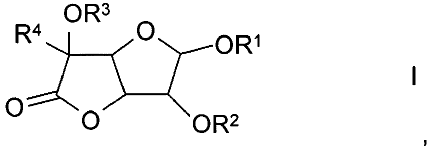
where R¹ and R² each, independently of one another, denote aryl,
a straight-chain or branched alkyl group having 2 to 30 C atoms, which may be substituted by aryl, or
a straight-chain or branched alkenyl group having 1 to 30 C atoms, containing one or more double bonds, which may be substituted by aryl,
where either R¹ or R² may denote H,
where R¹ and R² together may also form an unsubstituted or substituted five-membered ring, which may be substituted
a) by at least one straight-chain or branched alkyl group having 1 to 30 C atoms and/or
b) by at least one aryl group having 6 to 12 C atoms and/or
c) by at least one straight-chain or branched alkenyl group having 2 to 30 C atoms containing one or more double bonds,
where the alkyl group having 1 to 30 C atoms and/or the alkenyl group having 1 to 30 C atoms may be substituted by aryl and/or the aryl group having 6 to 12 C atoms may be substituted by alkyl, OH, Oalkyl, NH₂, NH-alkyl, N(alkyl)₂, C(O)alkyl, O-C(O)alkyl and/or C(O)-Oalkyl,
R³ denotes H or a straight-chain or branched alkyl group having 1 to 30 C atoms and
R⁴ denotes H or OR³,
where
aryl denotes an aryl group having 6 to 12 C atoms, which may optionally be substituted by alkyl, OH, Oalkyl, NH₂, NH-alkyl, N(alkyl)₂, C(O)alkyl, O-C(O)alkyl or C(O)-Oalkyl, and
alkyl denotes a straight-chain or branched alkyl group having 1 to 30 C atoms,
and/or salts, tautomers, stereoisomers and/or solvates thereof, including mixtures thereof in all ratios, for modulation of the colour shade achieved in the case of tanning with dihydroxyacetone or by the mixture or preparation comprising dihydroxyacetone.

4. Use of at least one compound of the formula I 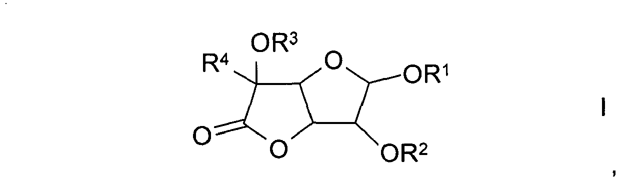
where R¹ and R² each, independently of one another, denote aryl,
a straight-chain or branched alkyl group having 1 to 30 C atoms, which may be substituted by aryl, or
a straight-chain or branched alkenyl group having 2 to 30 C atoms, containing one or more double bonds, which may be substituted by aryl,
where either R¹ or R² may denote H,
where R¹ and R² together may also form an unsubstituted or substituted five-membered ring, which may be substituted
a) by at least one straight-chain or branched alkyl group having 1 to 30 C atoms and/or
b) by at least one aryl group having 6 to 12 C atoms and/or
c) by at least one straight-chain or branched alkenyl group having 2 to 30 C atoms containing one or more double bonds,
where the alkyl group having 1 to 30 C atoms and/or the alkenyl group having 1 to 30 C atoms may be substituted by aryl and/or the aryl group having 6 to 12 C atoms may be substituted by alkyl, OH, Oalkyl, NH₂, NH-alkyl, N(alkyl)₂, C(O)alkyl, O-C(O)alkyl and/or C(O)-Oalkyl,
R³ denotes H or a straight-chain or branched alkyl group having 1 to 30 C atoms and
R⁴ denotes H or OR³,
where
aryl denotes an aryl group having 6 to 12 C atoms, which may optionally be substituted by alkyl, OH, Oalkyl, NH₂, NH-alkyl, N(alkyl)₂, C(O)alkyl, O-C(O)alkyl or C(O)-Oalkyl, and
alkyl denotes a straight-chain or branched alkyl group having 1 to 30 C atoms,
and/or salts, tautomers, stereoisomers and/or solvates thereof, including mixtures thereof in all ratios, as contrast-reduction agent.

5. Use of at least one compound of the formula I 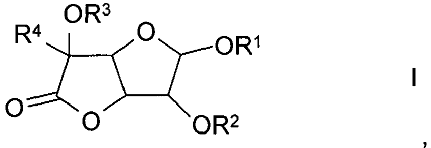
where R¹ and R² each, independently of one another, denote aryl,
a straight-chain or branched alkyl group having 1 to 30 C atoms, which may be substituted by aryl, or
a straight-chain or branched alkenyl group having 2 to 30 C atoms, containing one or more double bonds, which may be substituted by aryl,
where either R¹ or R² may denote H,
where R¹ and R² together may also form an unsubstituted or substituted five-membered ring, which may be substituted
g) by at least one straight-chain or branched alkyl group having 1 to 30 C atoms and/or
h) by at least one aryl group having 6 to 12 C atoms and/or
i) by at least one straight-chain or branched alkenyl group having 2 to 30 C atoms containing one or more double bonds,
where the alkyl group having 1 to 30 C atoms and/or the alkenyl group having 1 to 30 C atoms may be substituted by aryl and/or the aryl group having 6 to 12 C atoms may be substituted by alkyl, OH, Oalkyl, NH₂, NH-alkyl, N(alkyl)₂, C(O)alkyl, O-C(O)alkyl and/or C(O)-Oalkyl,
R³ denotes H or a straight-chain or branched alkyl group having 1 to 30 C atoms and
R⁴ denotes H or OR³,
where
aryl denotes an aryl group having 6 to 12 C atoms, which may optionally be substituted by alkyl, OH, Oalkyl, NH₂, NH-alkyl, N(alkyl)₂, C(O)alkyl, O-C(O)alkyl or C(O)-Oalkyl, and
alkyl denotes a straight-chain or branched alkyl group having 1 to 30 C atoms,
and/or salts, tautomers, stereoisomers and/or solvates thereof, including mixtures thereof in all ratios, for colouring keratin-containing fibres.

6. Use according to one or more of Claims 1 to 5, **characterised in that** the substituent R⁴ in the compound of the formula I denotes H.

7. Use according to one or more of Claims 1 to 6, **characterised in that** the substituent R³ in the compound of the formula I denotes H.

8. Use according to one or more of Claims 1 to 7, **characterised in that** the substituents R¹ and R² in the compound of the formula I together form a five-membered ring, which may be substituted by at least one straight-chain or branched alkyl group having 1 to 10 C atoms
and/or may be substituted by at least one straight-chain or branched alkenyl group having 1 to 10 C atoms containing one or more double bonds, which may be substituted by at least one phenyl group, where the phenyl group may be substituted by alkyl, OH, Oalkyl, C(O)alkyl, O-C(O)alkyl and/or C(O)-Oalkyl
and/or may be substituted by a phenyl group, where the phenyl group may be substituted by alkyl, OH, Oalkyl, C(O)alkyl, O-C(O)alkyl and/or C(O)-Oalkyl.

9. Use according to one or more of Claims 1 to 5, **characterised in that** the compound of the formula I is selected from compounds (Ia) to (Im)

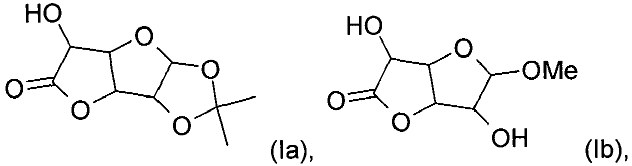

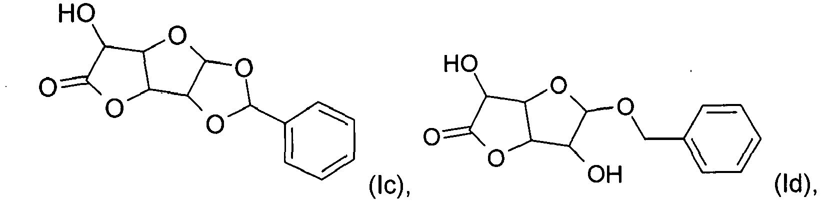

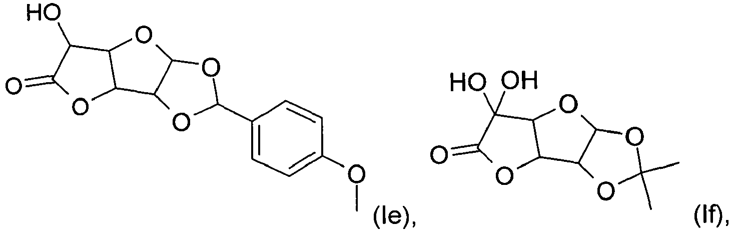

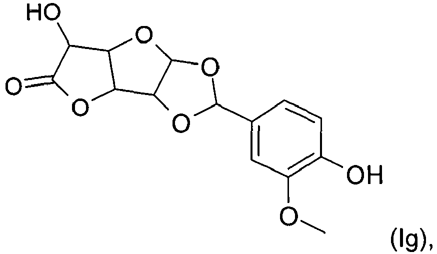

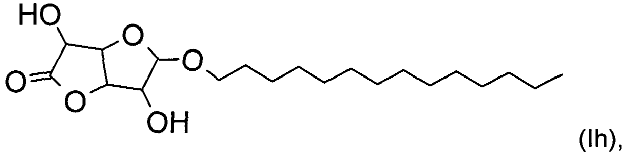

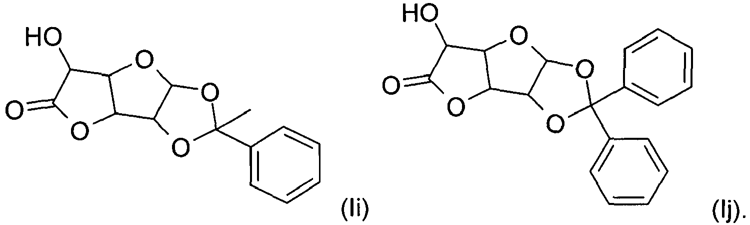

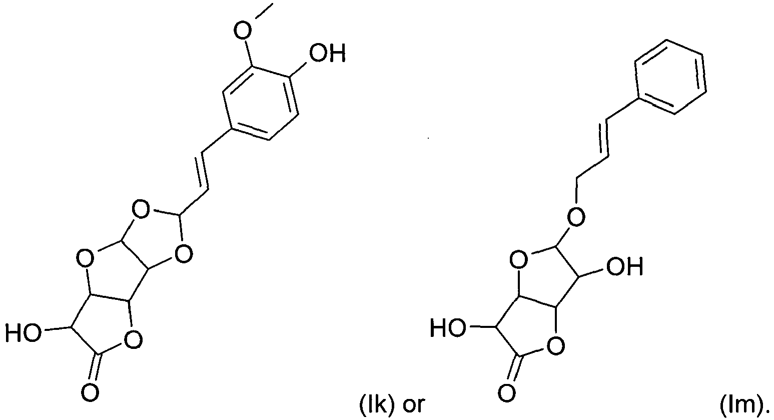

10. Preparation comprising dihydroxyacetone and at least one compound of the formula I according to one or more of Claims 1 to 9 and/or salts, tautomers, stereoisomers and/or solvates thereof, including mixtures thereof in all ratios.

11. Preparation according to Claim 10, **characterised in that** the at least one compound of the formula I is present in an amount of 0.01 to 20% by weight, based on the total amount of the preparation.

12. Preparation according to Claim 10 or 11, **characterised in that** a vehicle which is suitable for cosmetic, pharmaceutical, dermatological preparations is present.

13. Process for the preparation of a preparation according to Claim 12, **characterised in that** the at least one compound of the formula I is mixed together with at least one vehicle which is suitable for cosmetic, pharmaceutical, dermatological preparations and optionally assistants and/or fillers or self-tanning substances, and dihydroxyacetone is added.

14. Composition for colouring keratin-containing fibres, **characterised in that** at least one compound of the formula I according to one or more of Claims 5 to 9 and/or salts, tautomers, stereoisomers and/or solvates thereof, including mixtures thereof in all ratios, is present.

15. Composition according to Claim 14, **characterised in that** a further direct dye or at least one oxidation dye precursor is present.

16. Compounds of the formulae 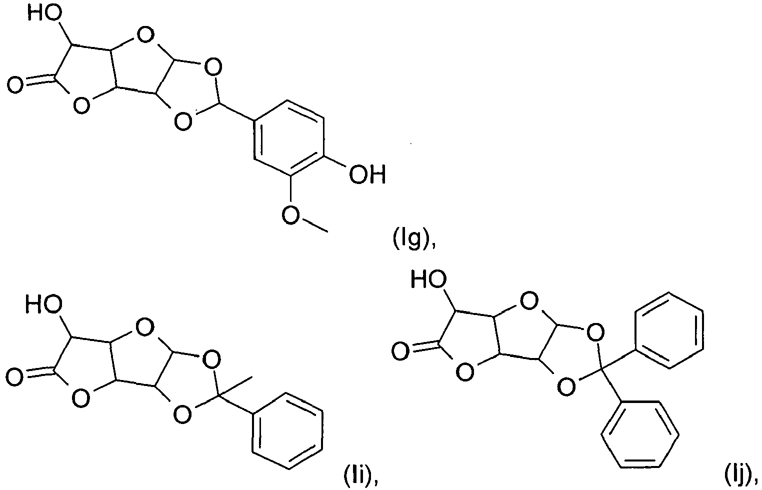 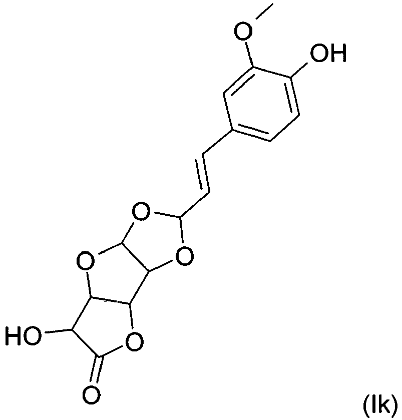 and

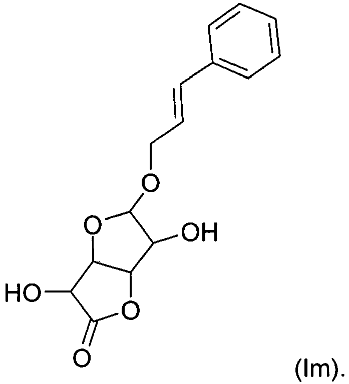

## Revendications

1. Utilisation d'au moins un composé de formule I 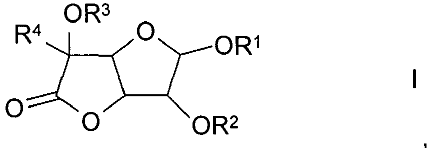
dans lequel R¹ et R² désignent chacun, indépendamment l'un de l'autre, aryle,
un groupement alkyle à chaîne linéaire ou ramifiée ayant de 1 à 30 atomes de C, pouvant être substitué par aryle, ou
un groupement alcényle à chaîne linéaire ou ramifiée ayant de 2 à 30 atomes de C, contenant une ou plusieurs doubles liaisons, pouvant être substitué par aryle,
où l'un ou l'autre parmi R¹ ou R² peut désigner H,
où R¹ et R² peuvent aussi former ensemble un cycle de cinq chaînons non substitué ou substitué, pouvant être substitué
a) par au moins un groupement alkyle à chaîne linéaire ou ramifiée ayant de 1 à 30 atomes de C et/ou
b) par au moins un groupement aryle ayant de 6 à 12 atomes de C et/ou
c) par au moins un groupement alcényle à chaîne linéaire ou ramifiée ayant de 2 à 30 atomes de C contenant une ou plusieurs doubles liaisons,
où le groupement alkyle ayant de 1 à 30 atomes de C et/ou le groupement alcényle ayant de 1 à 30 atomes de C peut être substitué par aryle et/ou le groupement aryle ayant de 6 à 12 atomes de C peut être substitué par alkyle, OH, Oalkyle, NH₂, NH-alkyle, N(alkyl)₂, C(O)alkyle, O-C(O)alkyle et/ou C(O)-Oalkyle,
R³ désigne H ou un groupement alkyle à chaîne linéaire ou ramifiée ayant de 1 à 30 atomes de C et
R⁴ désigne H ou OR³,
où
aryle désigne un groupement aryle ayant de 6 à 12 atomes de C, pouvant éventuellement être substitué par alkyle, OH, Oalkyle, NH₂, NH-alkyle, N(alkyl)₂, C(O)alkyle, O-C(O)alkyle ou C(O)-Oalkyle, et
alkyle désigne un groupement alkyle à chaîne linéaire ou ramifiée ayant de 1 à 30 atomes de C,
et/ou les sels, tautomères, stéréoisomères et/ou solvats de celui-ci, y compris des mélanges de ceux-ci en toutes proportions, comme substance auto-bronzante.

2. Utilisation d'au moins un composé de formule I 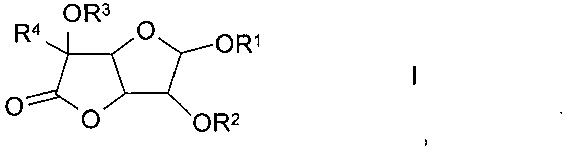
dans lequel R¹ et R² désignent chacun, indépendamment l'un de l'autre, aryle,
un groupement alkyle à chaîne linéaire ou ramifiée ayant de 1 à 30 atomes de C, pouvant être substitué par aryle, ou
un groupement alcényle à chaîne linéaire ou ramifiée ayant de 2 à 30 atomes de C, contenant une ou plusieurs doubles liaisons, pouvant être substitué par aryle,
où l'un ou l'autre parmi R¹ ou R² peut désigner H,
où R¹ et R² peuvent aussi former ensemble un cycle de cinq chaînons non substitué ou substitué, pouvant être substitué
a) par au moins un groupement alkyle à chaîne linéaire ou ramifiée ayant de 1 à 30 atomes de C et/ou
b) par au moins un groupement aryle ayant de 6 à 12 atomes de C et/ou
c) par au moins un groupement alcényle à chaîne linéaire ou ramifiée ayant de 2 à 30 atomes de C contenant une ou plusieurs doubles liaisons,
où le groupement alkyle ayant de 1 à 30 atomes de C et/ou le groupement alcényle ayant de 1 à 30 atomes de C peut être substitué par aryle et/ou le groupement aryle ayant de 6 à 12 atomes de C peut être substitué par alkyle, OH, Oalkyle, NH₂, NH-alkyle, N(alkyl)₂, C(O)alkyle, O-C(O)alkyle et/ou C(O)-Oalkyle,
R³ désigne H ou un groupement alkyle à chaîne linéaire ou ramifiée ayant de 1 à 30 atomes de C et
R⁴ désigne H ou OR³,
où
aryle désigne un groupement aryle ayant de 6 à 12 atomes de C, pouvant éventuellement être substitué par alkyle, OH, Oalkyle, NH₂, NH-alkyle, N(alkyl)₂, C(O)alkyle, O-C(O)alkyle ou C(O)-Oalkyle, et
alkyle désigne un groupement alkyle à chaîne linéaire ou ramifiée ayant de 1 à 30 atomes de C,
et/ou les sels, tautomères, stéréoisomères et/ou solvats de celui-ci, y compris des mélanges de ceux-ci en toutes proportions, comme améliorateur de bronzage pour la dihydroxyacétone ou pour un mélange de substances auto-bronzantes comprenant de la dihydroxyacétone.

3. Utilisation d'au moins un composé de formule I 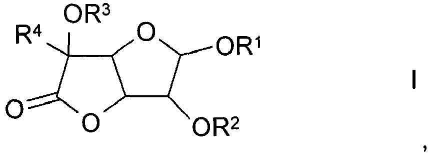
dans lequel R¹ et R² désignent chacun, indépendamment l'un de l'autre, aryle,
un groupement alkyle à chaîne linéaire ou ramifiée ayant de 2 à 30 atomes de C, pouvant être substitué par aryle, ou
un groupement alcényle à chaîne linéaire ou ramifiée ayant de 1 à 30 atomes de C, contenant une ou plusieurs doubles liaisons, pouvant être substitué par aryle,
où l'un ou l'autre parmi R¹ ou R² peut désigner H,
où R¹ et R² peuvent aussi former ensemble un cycle de cinq chaînons non substitué ou substitué, pouvant être substitué
a) par au moins un groupement alkyle à chaîne linéaire ou ramifiée ayant de 1 à 30 atomes de C et/ou
b) par au moins un groupement aryle ayant de 6 à 12 atomes de C et/ou
c) par au moins un groupement alcényle à chaîne linéaire ou ramifiée ayant de 2 à 30 atomes de C contenant une ou plusieurs doubles liaisons,
où le groupement alkyle ayant de 1 à 30 atomes de C et/ou le groupement alcényle ayant de 1 à 30 atomes de C peut être substitué par aryle et/ou le groupement aryle ayant de 6 à 12 atomes de C peut être substitué par alkyle, OH, Oalkyle, NH₂, NH-alkyle, (alkyl)₂, C(O)alkyle, O-C(O)alkyle et/ou C(O)-Oalkyle,
R³ désigne H ou un groupement alkyle à chaîne linéaire ou ramifiée ayant de 1 à 30 atomes de C et
R⁴ désigne H ou OR³,
où
aryle désigne un groupement aryle ayant de 6 à 12 atomes de C, pouvant éventuellement être substitué par alkyle, OH, Oalkyle, NH₂, NH-alkyle, N(alkyl)₂, C(O)alkyle, O-C(O)alkyle ou C(O)-Oalkyle, et
alkyle désigne un groupement alkyle à chaîne linéaire ou ramifiée ayant de 1 à 30 atomes de C,
et/ou les sels, tautomères, stéréoisomères et/ou solvats de celui-ci, y compris des mélanges de ceux-ci en toutes proportions, pour la modulation de la nuance de couleur obtenue dans le cas d'un bronzage par de la dihydroxyacétone ou par le mélange ou la préparation comprenant de la dihydroxyacétone.

4. Utilisation d'au moins un composé de formule I 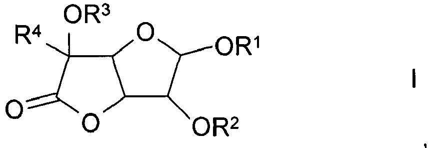
dans lequel R¹ et R² désignent chacun, indépendamment l'un de l'autre, aryle,
un groupement alkyle à chaîne linéaire ou ramifiée ayant de 1 à 30 atomes de C, pouvant être substitué par aryle, ou
un groupement alcényle à chaîne linéaire ou ramifiée ayant de 2 à 30 atomes de C, contenant une ou plusieurs doubles liaisons, pouvant être substitué par aryle,
où l'un ou l'autre parmi R¹ ou R² peut désigner H,
où R¹ et R² peuvent aussi former ensemble un cycle de cinq chaînons non substitué ou substitué, pouvant être substitué
a) par au moins un groupement alkyle à chaîne linéaire ou ramifiée ayant de 1 à 30 atomes de C et/ou
b) par au moins un groupement aryle ayant de 6 à 12 atomes de C et/ou
c) par au moins un groupement alcényle à chaîne linéaire ou ramifiée ayant de 2 à 30 atomes de C contenant une ou plusieurs doubles liaisons,
où le groupement alkyle ayant de 1 à 30 atomes de C et/ou le groupement alcényle ayant de 1 à 30 atomes de C peut être substitué par aryle et/ou le groupement aryle ayant de 6 à 12 atomes de C peut être substitué par alkyle, OH, Oalkyle, NH₂, NH-alkyle, N(alkyl)₂, C(O)alkyle, O-C(O)alkyle et/ou C(O)-Oalkyle,
R³ désigne H ou un groupement alkyle à chaîne linéaire ou ramifiée ayant de 1 à 30 atomes de C et
R⁴ désigne H ou OR³,
où
aryle désigne un groupement aryle ayant de 6 à 12 atomes de C, pouvant éventuellement être substitué par alkyle, OH, Oalkyle, NH₂, NH-alkyle, N(alkyl)₂, C(O)alkyle, O-C(O)alkyle ou C(O)-Oalkyle, et
alkyle désigne un groupement alkyle à chaîne linéaire ou ramifiée ayant de 1 à 30 atomes de C,
et/ou les sels, tautomères, stéréoisomères et/ou solvats de celui-ci, y compris des mélanges de ceux-ci en toutes proportions, comme agent de réduction de contraste.

5. Utilisation d'au moins un composé de formule I 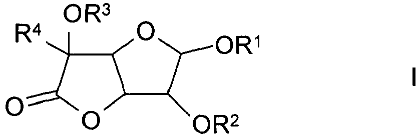
dans lequel R¹ et R² désignent chacun, indépendamment l'un de l'autre, aryle,
un groupement alkyle à chaîne linéaire ou ramifiée ayant de 1 à 30 atomes de C, pouvant être substitué par aryle, ou
un groupement alcényle à chaîne linéaire ou ramifiée ayant de 2 à 30 atomes de C, contenant une ou plusieurs doubles liaisons, pouvant être substitué par aryle,
où l'un ou l'autre parmi R¹ ou R² peut désigner H,
où R¹ et R² peuvent aussi former ensemble un cycle de cinq chaînons non substitué ou substitué, pouvant être substitué
g) par au moins un groupement alkyle à chaîne linéaire ou ramifiée ayant de 1 à 30 atomes de C et/ou
h) par au moins un groupement aryle ayant de 6 à 12 atomes de C et/ou
i) par au moins un groupement alcényle à chaîne linéaire ou ramifiée ayant de 2 à 30 atomes de C contenant une ou plusieurs doubles liaisons,
où le groupement alkyle ayant de 1 à 30 atomes de C et/ou le groupement alcényle ayant de 1 à 30 atomes de C peut être substitué par aryle et/ou le groupement aryle ayant de 6 à 12 atomes de C peut être substitué par alkyle, OH, Oalkyle, NH₂, NH-alkyle, N(alkyl)₂, C(O)alkyle, 0-C(O)alkyle et/ou C(O)-Oalkyle,
R³ désigne H ou un groupement alkyle à chaîne linéaire ou ramifiée ayant de 1 à 30 atomes de C et
R⁴ désigne H ou OR³,
où
aryle désigne un groupement aryle ayant de 6 à 12 atomes de C, pouvant éventuellement être substitué par alkyle, OH, Oalkyle, NH₂, NH-alkyle, N(alkyl)₂, C(O)alkyle, O-C(O)alkyle ou C(O)-Oalkyle, et
alkyle désigne un groupement alkyle à chaîne linéaire ou ramifiée ayant de 1 à 30 atomes de C,
et/ou les sels, tautomères, stéréoisomères et/ou solvats de celui-ci, y compris des mélanges de ceux-ci en toutes proportions, pour la coloration de fibres kératiniques.

6. Utilisation selon l'une ou plusieurs parmi les revendications 1 à 5, **caractérisée en ce que** le substituant R⁴ dans le composé de formule I désigne H.

7. Utilisation selon l'une ou plusieurs parmi les revendications 1 à 6, **caractérisée en ce que** le substituant R³ dans le composé de formule I désigne H.

8. Utilisation selon l'une ou plusieurs parmi les revendications 1 à 7, **caractérisée en ce que** le substituants R¹ et R² dans le composé de formule I forment ensemble un cycle de cinq chaînons, pouvant être substitué par au moins un groupement alkyle à chaîne linéaire ou ramifiée ayant de 1 à 10 atomes de C
et/ou pouvant être substitué par au moins un groupement alcényle à chaîne linéaire ou ramifiée ayant de 1 à 10 atomes de C contenant une ou plusieurs doubles liaisons, pouvant être substitué par au moins un groupement phényle, où le groupement phényle peut être substitué par alkyle, OH, Oalkyle, C(O)alkyle, O-C(O)alkyle et/ou C(O)-Oalkyle et/ou peut être substitué par un groupement phényle, où le groupement phényle peut être substitué par alkyle, OH, Oalkyle, C(O)alkyle, O-C(O)alkyle et/ou C(O)-Oalkyle.

9. Utilisation selon l'une ou plusieurs parmi les revendications 1 à 5, **caractérisée en ce que** le composé de formule I est choisi parmi les composés (la) à (Im)

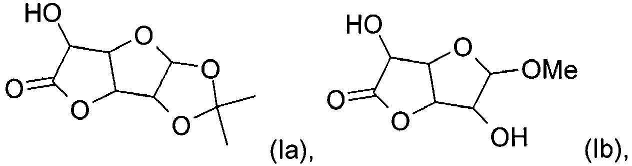

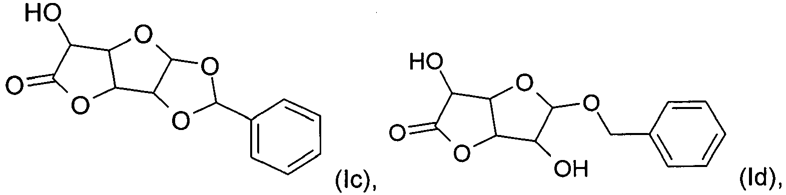

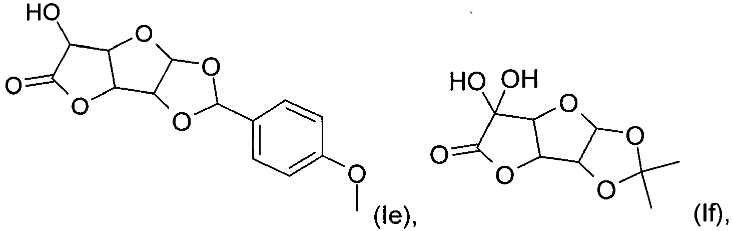

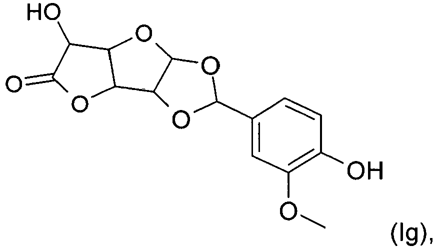

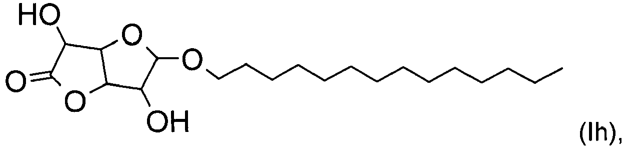

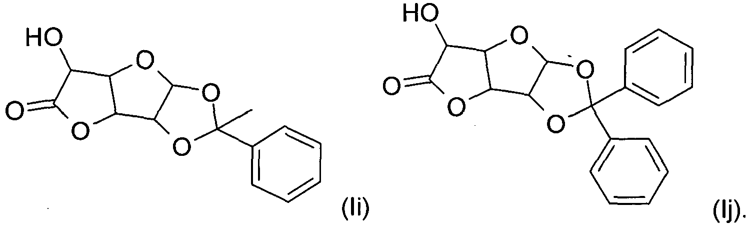

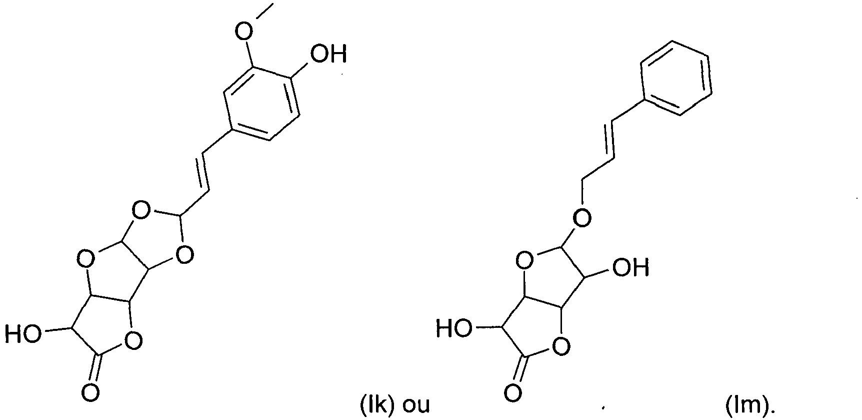

10. Préparation comprenant de la dihydroxyacétone et au moins un composé de formule I selon l'une ou plusieurs parmi les revendications 1 à 9 et/ou des sels, tautomères, stéréoisomères et/ou solvats de celui-ci, y compris des mélanges de ceux-ci en toutes proportions.

11. Préparation selon la revendication 10, **caractérisée en ce que** le au moins un composé de formule I est présent selon une quantité allant de 0,01 à 20% en poids, sur la base de la quantité totale de la préparation.

12. Préparation selon la revendication 10 ou 11, **caractérisée en ce qu'**un véhicule qui est convenable pour des préparations cosmétiques, pharmaceutiques, dermatologiques est présent.

13. Procédé de préparation d'une préparation selon la revendication 12, **caractérisé en ce que** le au moins un composé de formule I est mélangé avec au moins un véhicule qui est convenable pour des préparations cosmétiques, pharmaceutiques, dermatologiques et éventuellement des auxiliaires et/ou des charges ou des substances auto-bronzantes, et on ajoute de la dihydroxyacétone.

14. Composition destinée à la coloration de fibres kératiniques, **caractérisée en ce qu'**au moins un composé de formule I selon l'une ou plusieurs parmi les revendications 5 à 9 et/ou les sels, tautomères, stéréoisomères et/ou solvats de celui-ci, y compris des mélanges de ceux-ci en toutes proportions, est présent.

15. Composition selon la revendication 14, **caractérisée en ce qu'**un colorant direct supplémentaire ou au moins un précurseur de colorant d'oxydation est présent.

16. Composés de formules 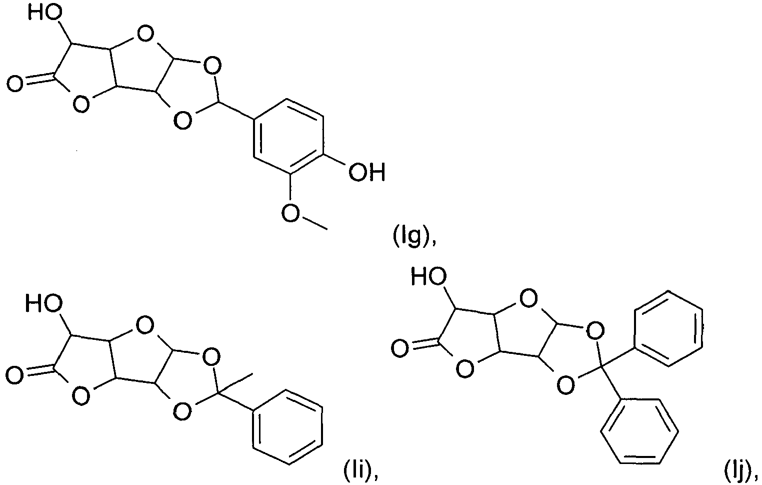 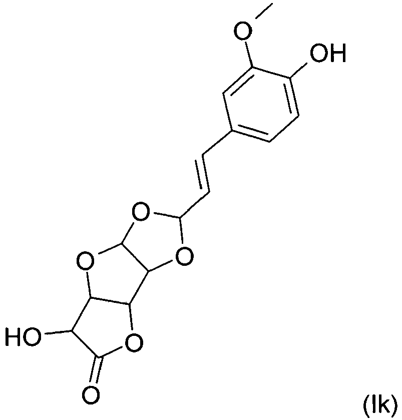 et
